(19) Europäisches Patentamt
European Patent Office
Office européen des brevets

(11) Veröffentlichungsnummer: **0 325 160 A2**

# (12) EUROPÄISCHE PATENTANMELDUNG

(21) Anmeldenummer: **89100451.7**

(22) Anmeldetag: **12.01.89**

(51) Int. Cl.⁴: **C07K 5/06 , A61K 37/02**

Patentansprüche für folgende Vertragsstaaten: ES + GR.

(30) Priorität: **21.01.88 DE 3801587**

(43) Veröffentlichungstag der Anmeldung: **26.07.89 Patentblatt 89/30**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Rüger, Wolfgang, Dr.**
**Parkstrasse 10**
**D-6233 Kelkheim (Taunus)(DE)**
Erfinder: **Urbach, Hansjörg, Dr.**
**Le Lavandoustrasse 41**
**D-6242 Kronberg/Taunus(DE)**
Erfinder: **Becker, Reinhard, Dr.**
**Humboldtstrasse 17**
**D-6200 Wiesbaden(DE)**
Erfinder: **Hock, Franz, Dr.**
**Altstadt 19**
**D-6110 Dieburg(DE)**

## (54) Neue Aminosäureglyceride, Verfahren zu ihrer Herstellung, sie enthaltende Arzneimittel und deren Verwendung.

(57) Die Erfindung betrifft Aminosäureester der Formel I

$$R^3OOC - \underset{R^4}{\overset{*}{CH}} - \underset{R^5}{N} - \underset{O}{\overset{}{C}} - \underset{R^1}{\overset{*}{CH}} - NH - \underset{COOR_2}{\overset{*}{CH}} - (CH_2)_n - R \qquad (I)$$

worin n = 1 oder 2 ist, R, $R^1$, $R^2$ und $R^3$ Wasserstoff oder einen definierten Rest bedeuten, $R^4$ zusammen mit $R^5$ und den sie tragenden Atomen ein heterocyclisches Ringsystem bilden, Verfahren zu ihrer Herstellung, sie enthaltende Mittel und deren Verwendung.

EP 0 325 160 A2

# Neue Aminosäureglyceride, Verfahren zu ihrer Herstellung, sie enthaltende Arzneimittel und deren Verwendung

Die Erfindung betrifft Aminosäureester der Formel I

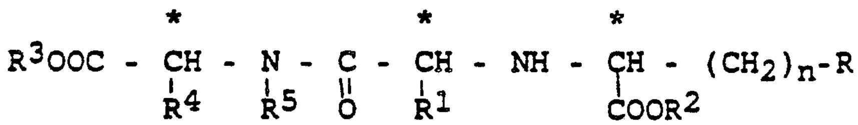

(I)

in welcher

n = 1 oder 2 ist,

R = Wasserstoff,

einen gegebenenfalls substituierten aliphatischen Rest mit 1 - 21 C-Atomen,

einen gegebenenfalls substituierten alicyclischen Rest mit 3 - 20 C-Atomen,

einen gegebenenfalls substituierten aromatischen Rest mit 6 - 12 C-Atomen,

einen gegebenenfalls substituierten araliphatischen Rest mit 7 - 32 C-Atomen,

einen gegebenenfalls substituierten alicyclisch-aliphatischen Rest mit 7 - 14 C-Atomen,

einen gegebenenfalls substituierten heteroaromatischen oder heteroaromatisch- ($C_1$-$C_8$)-aliphatischen Rest mit 5 - 12 Ringatomen, oder

einen Rest $OR^a$ oder $SR^a$ bedeutet, worin

$R^a$ für einen gegebenenfalls substituierten aliphatischen Rest mit 1 - 4 C-Atomen, für einen gegebenenfalls substituierten aromatischen Rest mit 6 - 12 C-Atomen oder einen gegebenenfalls substituierten heteroaromatischen Rest mit 5 - 12 Ringatomen steht,

$R^1$ Wasserstoff,

einen gegebenenfalls substituierten aliphatischen Rest mit 1 - 21 C-Atomen,

einen gegebenenfalls substituierten alicyclischen Rest mit 3 - 20 C-Atomen,

einen gegebenenfalls substituierten alicyclischaliphatischen Rest mit 4 - 20 C-Atomen,

einen gegebenenfalls substituierten aromatischen Rest mit 6 - 12 C-Atomen,

einen gegebenenfalls substituierten araliphatischen Rest mit 7 - 32 C-Atomen,

einen gegebenenfalls substituierten heteroaromatischen oder heteroaromatisch-($C_1$-$C_8$)-aliphatischen Rest mit 5 - 12 Ringatomen oder,

falls nicht von vorstehenden Definitionen schon umfaßt, die erforderlichenfalls geschützte Seitenkette einer natürlich vorkommenden α-Aminosäure bedeutet,

$R^2$ und $R^3$ gleich oder verschieden sind und Wasserstoff, einen gegebenenfalls substituierten aliphatischen Rest mit 1 - 21 C-Atomen, einen gegebenenfalls substituierten alicyclischen Rest mit 3 - 20 C-Atomen,

einen gegebenenfalls substituierten aromatischen Rest mit 6 - 12 C-Atomen,

einen gegebenenfalls substituierten araliphatischen Rest mit 7 - 32 C-Atomen,

einen Rest der Formel

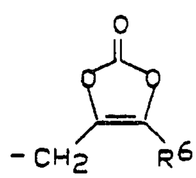

worin

$R^6$ Wasserstoff, ein aliphatischen Rest mit 1 - 6 C-Atomen oder ein gegebenenfalls substituierter aromatischer Rest mit 6 - 12 C-Atomen ist,

einen Rest der Formel

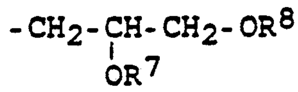 oder 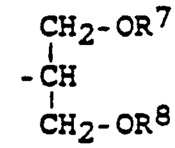

worin

$R^7$ und $R^8$ gleich oder verschieden unabhängig voneinander Wasserstoff, einen gegebenenfalls substituierten unverzweigten oder verzweigten, gesättigten oder ungesättigten Alkylrest mit 1 - 23 C-Atomen oder einen gegebenenfalls substituierten, unverzweigten oder verzweigten, gesättigten oder ungesättigten Acylrest mit 1 - 23 C-Atomen bedeuten,

bedeuten,

wobei mindestens einer der Reste $R^2$ oder $R^3$ einer Rest der Formel

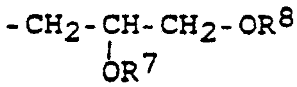 oder 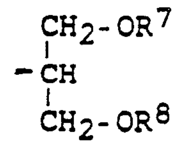

bedeutet, und

$R^4$ and $R^5$ zusammen mit den sie tragenden Atomen ein mono-, bi-, oder tricyclisches heterocyclisches Ringsystem mit 3 bis 15 Ring-C-Atomen bilden,

sowie deren physiologisch verträgliche Salze mit Säure und Basen.

Ein gegebenenfalls substituierter, unverzweigter oder verzweigter, gesättigter oder ungesättigter Acylrest ist vorzugsweise ein Rest einer aliphatischen Carbonsäure, insbesondere gesättigtes oder ungesättigtes Alkanoyl, wie z.B. Formyl, Acetyl, Propionyl, Butyryl, Valeryl, Isovaleryl, Pivaloyl, Lauroyl, Myristoyl, Palmitoyl, Stearoyl, Acryloyl, Propioloyl, Methacryloyl, Crotonoyl, Isocrotonoyl, OLeoyl, Elaidoyl, Acetoacetyl, Behenoyl, Caprinoyl, Caproyl, Capryloyl, Sorboyl, Lävulinoyl, Lignoceroyl, Önanthoyl, Pelargonoyl, Ricinoloyl oder Tigloyl. Bevorzugt sind mittel- oder längerkettige Fettsäureester, wie sie beispielsweise in natürlichen Triglyceriden vorkommen. Unter einem gegebenenfalls substituierten aliphatischen Rest versteht man einen aliphatischen acyclischen Rest, d.h. einen Rest mit einer offenen, geraden oder verzweigten Kohlenstoffkette, wie beispielsweise Alkyl, Alkenyl, Alkinyl und entsprechende mehrfach ungesättigte Reste. Er ist unsubstituiert oder, wie unten beispielsweise bei Carboxy, Carbamoyl, Aminoalkyl, Alkanoylaminoalkyl, Alkoxycarbonylaminoalkyl, Arylalkoxycarbonylaminoalkyl, Arylalkylaminoalkyl, Alkylaminoalkyl, Dialkylaminoalkyl, Alkylthioalkyl, Arylthioalkyl, Carboxyalkyl, Carbamoylalkyl, Alkoxycarbonylalkyl, Alkanoyloxyalkyl, Alkoxycarbonyloxyalkyl, Aroyloxyalkyl oder Aryloxycarbonyloxyalkyl beschrieben, monosubstituiert.

Ein gegebenenfalls substituierter alicyclischer Rest und der über eine offene Kohlenstoffkette gebundene entsprechende gegebenenfalls substituierte alicyclisch-aliphatische Rest ist ein vorzugsweise mono- bis pentacyclischer isocyclischer, nicht aromatischer Rest mit Einfach- oder unsymmetrisch verteilten Doppelbindungen, der auch verzweigt sein (d.h. offenkettige aliphatische Seitenketten tragen) kann, und der über ein Ring-C-Atom oder ein Seitenketten-C-Atom verknüpft ist. Er ist vorzugsweise unsubstituiert. Mehrere Ringe als Komponenten eines solchen Restes sind kondensiert, spiroverknüpft oder isoliert. Beispiele für solche Reste sind Cycloalkyl, Cycloalkenyl, Cycloalkylalkyl, Bicycloalkyl, Tricycloalkyl und von mono-, bi- oder oligocyclischen Terpenen abgeleitete Reste, wie Menthyl, Isomenthyl, Bornyl, Caranyl, Epibornyl, Epiisobornyl, Isobornyl, Norbornyl, Neomenthyl, Neoisomenthyl, Pinanyl, Thujanyl; sie sind vorzugsweise unsubstituiert (aliphatische Seitenketten sind nach vorliegender Definition keine Substituenten).

Ein gegebenenfalls substituierter aromatischer Rest ist vorzugsweise Aryl wie Phenyl, Biphenylyl oder Naphthyl, das gegebenenfalls wie unten bei Aryl angegeben mono-, di- oder trisubstituiert ist. Von Aryl abgeleitete Reste, wie Aralkyl, Aryloxy, Arylthio oder Aroyl, vorzugsweise Benzoyl, können wie Aryl substituiert sein.

Ein gegebenenfalls substituierter heteroaromatischer Rest ist vorzugsweise ein aromatischer mono- bzw. bicyclischer Heterocyclen-Rest mit 5 bis 7 bzw. 8 bis 12, bevorzugt bis 10 Ringatomen, wovon 1 bis 2 Ringatome Schwefel- oder Sauerstoffatome und/oder wovon 1 bis 4 Ringatome Stickstoffatome darstellen, wie beispielsweise Thienyl, Benzo[b]thienyl, Furyl, Pyranyl, Benzofuryl, Pyrrolyl, Imidazolyl, Pyrazolyl, Pyridyl, Pyrimidinyl, Pyridazinyl, Indazolyl, Isoindolyl, Indolyl, Purinyl, Chinolizinyl, Isochinolinyl, Phthalazinyl, Naphthyridinyl, Chinoxalinyl, Chinazolyl, Cinnolinyl, Pteridinyl, Oxazolyl, Isoxazolyl, Thiazolyl oder Isothiazolyl. Diese Rest können auch teilweise oder vollständig hydriert sein. Ein heteroaromatischer Rest und der entsprechende heteroaromatisch-aliphatische Rest kann wie unten definiert, substituiert sein.

Unter einem gegebenenfalls substituierten araliphatischen Rest werden insbesondere Phthalidyl und Arylalkyl, Diarylalkyl, Indanyl oder Fluorenyl verstanden, worin Aryl wie oben definiert ist und in der dort angegebenen Weise substituiert sein kann.

$R^4$ und $R^5$ können mit den diese tragenden Atomen ein mono-, bi- oder tricyclisches heterocyclisches Ringsystem mit 3 bis 15 Ring-C-Atomen bilden, das im Ring vorzugsweise bis zu 2 S-Atome und bis 2 N-Atome, insbesondere bis zu 1 S-Atom aufweist.

Als solche Ringsystem kommen insbesondere jene aus der folgenden Gruppe in Betracht: Tetrahydroisochinolin (A); Decahydroisochinolin (B); Octahydroindol (C); Octahydrocyclopenta[b]pyrrol (D); 2-Azaspiro[4.5]decan (E); 2-Azaspiro[4.4]-nonan (F); Spiro[(bicyclo[2.2.1]heptan)-2,3′-pyrrolidin] (G); Spiro[-(bicyclo[2.2.2]octan)-2,3′-pyrrolidin] (H); 2-Azatricyclo[4.3.0.1$^{6,9}$]decan (I); Decahydrocyclohepta[b]pyrrol (J); Hexahydrocyclopropa[b]pyrrol (K); Octahydroisoindol (L); Octahydrocyclopenta[c]pyrrol (M) 2,3,3a,4,5,7a-Hexahydroindol (N); 1,2,3,3a,4,6a-Hexahydrocyclopenta[b]pyrrol (O); Pyrrolidin (P); Indolin (Q); Thiazolidin (R), die alle gegebenenfalls substituiert sein können.

Pyrrolidin (P) und Thiazolidin (R) können z.B. durch ($C_6$-$C_{12}$)-Aryl (Phenyl, 2-Hydroxyphenyl, u.a.), ($C_6$-$C_{12}$)-Arylmercapto (wie Phenylmercapto) oder ($C_3$-$C_7$)-Cycloalkyl (wie Cyclohexyl) monosubstituiert sein.

Tetrahydroisochinolin (A) kann z.B. im Arylteil bis zu 2 ($C_1$-$C_6$)-Alkoxyreste, vorzugsweise Methoxyreste tragen. Entsprechendes gilt für die anderen Ringsystem. Bevorzugt sind jedoch die unsubstituierten Systeme.

Die in Betracht kommenden cyclischen Aminosäureester weisen die folgenden Strukturformeln auf:

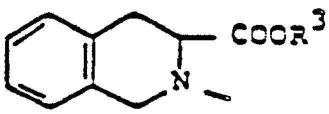

A

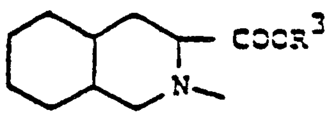

B

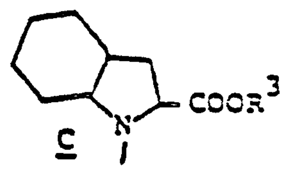

C

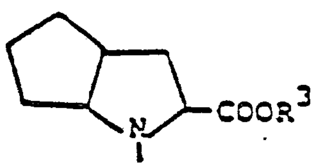

D

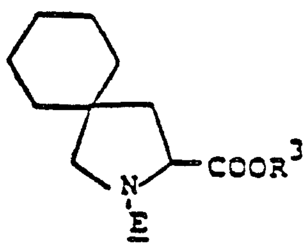

E

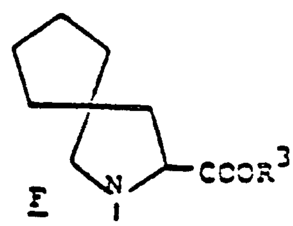

F

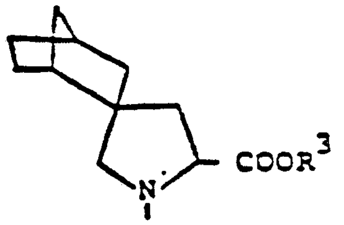
G

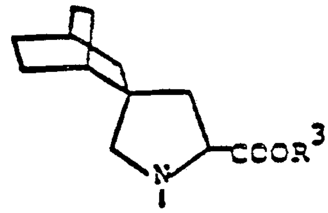
H

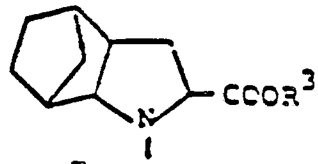
I

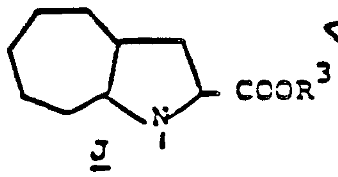
J

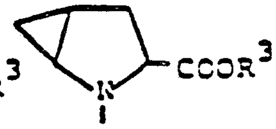
K

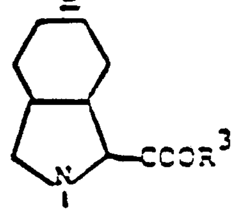
L

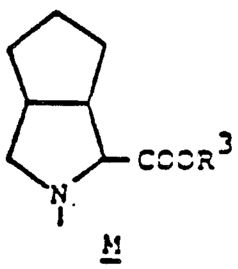
M

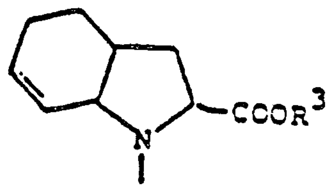
N

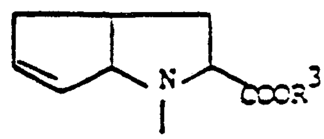
O

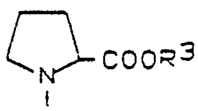
P

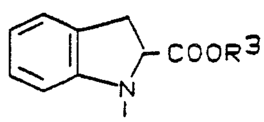
Q

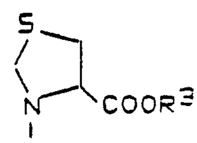
R

Besonders bevorzugt sind die Ringsystem A, C, D, H, O und P, in welchen das die $COOR^3$-Gruppe tragende C-Atom vorzugsweise die S-Konfiguration aufweist.

Natürlich vorkommende α-Aminosäuren sind beispielsweise Ala, Ser, Thr, Val, Leu, Ile, Asp, Asn, Glu, Gln, Arg, Lys, Hyl, Orn, Cit, Tyr, Phe, Trp und His.

Falls $R^1$ für eine Seitenkette einer geschützten natürlich vorkommenden α-Aminosäure steht, wie z.B geschütztes Ser, Thr, Asp, Asn, Glu, Gln, Arg, Lys, Hyl, Cys, Orn, Cit, Tyr, Trp oder His, sind als Schutzgruppen die in der Peptidchemie üblichen Gruppen bevorzugt (vgl. Houben-Weyl, Bd. XV/1 und XV/2). Im falle, daß $R^1$ die geschützte Lysin-Seitenkette bedeutet, werden die bekannten Amino-Schutzgruppen, insbesondere aber Z, Boc oder $(C_1$-$C_6)$-Alkanoyl bevorzugt. Als O-Schutzgruppen für Tyrosin kommen bevorzugt $(C_1$-$C_6)$-Alkyl, insbesondere Methyl oder Ethyl in Frage.

Die Verbindungen der Formel I weisen asymmetrische Kohlenstoffatome auf und können daher als Enantiomere und Diastereomere auftreten. Die Erfindung umfaßt sowohl die reinen Enantiomere als auch die Racemate.

Bei den Verbindungen, die mehrere chirale Atome besitzen, kommen alle möglichen Diastereomere als Racemate oder Enantiomere, oder Gemische verschiedener Diastereomere in Betracht. Die Racemate können nach gebräuchlichen Methoden, zum Beispiel durch Salzbildung mit optisch aktiven Säuren wie Camphersulfonsäure oder Di-benzoylweinsäure, fraktionierte Kristallisation und anschließende Freisetzung der Basen aus ihren Salzen, oder durch Derivatisierung mit geeigneten optisch aktiven Reagenzien, Trennung der diastereomeren Derivate durch fraktionierte Kristallisation oder Chromatographie an Kieselgel oder Aluminiumoxid und Rückspaltung in die Enantiomeren aufgetrennt werden. Die Diastereomeren können nach gebräuchlichen Methoden, wie fraktionierte Kristallisation oder Chromatographie an Säulen, getrennt werden.

Bevorzugt sind Verbindungen der Formel I, in welchen

a) n = 1 oder 2 ist;

b) R

1. Wasserstoff bedeutet;
2. Alkyl mit 1 - 18 C-Atomen bedeutet;
3. einen aliphatischen acyclischen Rest der Formel $C_aH_{2a-b+1})$ bedeutet, worin Doppelbindungen, falls ihre Zahl 1 übersteigt, nicht kumuliert sind, a für eine ganze Zahl 2 bis 18 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganz Zahl 3 bis 20 und d für eine gerade Zahl 0 bis (c-2) stehen;
5. Aryl mit 6 - 12 C-Atomen bedeutet,
das durch ($C_1$-$C_8$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Alkanoylamino, Methylendioxy, Carboxy, Cyano und/oder Sulfamoyl, mono-, di- oder trisubstituiert sein kann;
6. falls n = 2 ist, ($C_6$-$C_{12}$)-Aryl-($C_1$-$C_8$)-alkyl oder Di-($C_6$-$C_{12}$)-aryl-($C_1$-$C_8$)-alkyl bedeutet,
die im Arylteil jeweils wie unter b) 5. beschrieben substituiert sein können; oder
7. Alkoxy mit 1 - 4 C-Atomen,
8. Aryloxy mit 6- 12 C-Atomen,
das wie unter b) 5. beschrieben substituiert sein kann;
9. mono- bzw. bicyclisches Heteroaryloxy oder Heteroaryl- ($C_1$-$C_8$)-alkyl mit 5 - 7 bzw. 8 - 10 Ringatomen, wovon bis zu 9 Ringatome Kohlenstoff und 1 bis 2 Ringatome Schwefel oder Sauerstoff und/oder 1 bis 4 Ringatome Stickstoff darstellen, die im Heteroaryl wie unter b) 5. beschrieben substituiert sein können;
10. Amino-($C_1$-$C_8$)-alkyl;
11. ($C_1$-$C_4$)-Alkanoylamino-($C_1$-$C_8$)-alkyl;
12. ($C_7$-$C_{13}$)-Aroylamino-($C_1$-$C_8$)-alkyl;
13. ($C_1$-$C_4$)-Alkoxy-carbonylamino-($C_1$-$C_8$)-alkyl;
14. ($C_6$-$C_{12}$)-Aryl-($C_1$-$C_4$)-alkoxycarbonylamino-($C_1$-$C_8$)-alkyl;
15. ($C_6$-$C_{12}$)-Aryl-($C_1$-$C_4$)-alkylamino-($C_1$-$C_8$)-alkyl;
16. ($C_1$-$C_4$)-Alkylamino-($C_1$-$C_8$)-alkyl;
17. Di-($C_1$-$C_4$)-alkylamino-($C_1$-$C_8$)-alkyl;
18. Guanidino- ($C_1$-$C_8$)-alkyl;
19. Imidazolyl;
20. Indolyl;
21. ($C_1$-$C_4$)-Alkylthio;
22. falls n = 2 ist, ($C_1$-$C_4$)-Alkylthio-($C_1$-$C_8$)-alkyl;
23. ($C_6$-$C_{12}$)-Arylthio-($C_1$-$C_8$)-alkyl,
das im Arylteil wie unter b) 5. beschrieben, substituiert sein kann;
24. ($C_6$-$C_{12}$)-Aryl-($C_1$-$C_8$)-alkylthio,
das im Arylteil wie unter b) 5. beschrieben substituiert sein kann;
25. falls n = 2 ist, Carboxy-($C_1$-$C_8$)-alkyl;
26. Carboxy;
27. Carbamoyl;
28. falls n = 2 ist, Carbamoyl-($C_1$-$C_8$)-alkyl;
29. ($C_1$-$C_4$)-Alkoxy-carbonyl-($C_1$-$C_8$)-alkyl;
30. falls n = 2 ist, ($C_6$-$C_{12}$)-Aryloxy-($C_1$-$C_8$)-alkyl,
das im Arylteil wie unter b) 5. beschrieben substituiert sein kann; oder
31. ($C_6$-$C_{12}$)-Aryl-($C_1$-$C_8$)-alkoxy,
das im Arylteil wie unter b) 5. beschrieben substituiert sein kann, bedeutet;

c) $R^1$
1. Wasserstoff bedeutet;
2. Alkyl mit 1 - 18 C-Atomen bedeutet;
3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen; oder

5. Aryl mit 6 - 12 C-Atomen,
das wie unter b) 5. beschreiben substituiert sein kann;
6. ($C_6$-$C_{12}$)-Aryl-($C_1$-$C_8$)-alkyl oder ($C_7$-$C_{13}$)-Aroyl-($C_1$-$C_8$)-alkyl,
die beide im Arylteil wie unter b) 5. beschrieben substituiert sein können;
7. mono- bzw. bicyclisches, gegebenenfalls teilhydriertes Heteroaryl oder Heteroaryl-($C_1$-$C_8$)-alkyl mit 5 - 7 bzw. 8 - 10 Ringatomen, woven bis zu 9 Ringatome Kohlenstoff und 1 bis 2 Ringatome Schwefel- oder Sauerstoffatome und/oder 1 bis 4 Ringatome Stickstoffatome darstellen, die im Heteroaryl wie bei Aryl under b) 5. beschrieben substituiert sein können; oder
8. falls von c) 1. -7. noch nicht umfaßt, die gegebenenfalls geschützte Seitenkette einer natürlich verkommenden α-Aminosäure der Formel $R^1$-CH($NH_2$)-COOH bedeutet;

d) $R^2$ und $R^3$ gleich oder verschieden sind und
1. Wasserstoff bedeuten;
2. Alkyl mit 1 - 18 C-Atomen bedeuten;
3. einen aliphatischen acyclischen Rest der Formel $C_aH_{(2a-b+1)}$ bedeuten, worin Doppelbindungen, falls ihre Zahl 1 übersteigt, nicht kumuliert sind, a für eine ganze Zahl 2 bis 18 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeuten, der gegebenenfalls verzweigt ist, worin c für eine ganze Zahl 3 bis 20 und d für eine gerade Zahl 0 bis (c-2) stehen; oder
5. Di-($C_1$-$C_4$)-alkylamino-($C_1$-$C_8$)-alkyl;
6. ($C_1$-$C_5$)-Alkanoyloxy-($C_1$-$C_8$)-alkyl;
7. ($C_1$-$C_6$)-Alkoxy-carbonyloxy-($C_1$-$C_8$)-alkyl;
8. ($C_7$-$C_{13}$)-Aroyloxy-($C_1$-$C_8$)-alkyl;
9. ($C_6$-$C_{12}$)-Aryloxycarbonyloxy($C_1$-$C_8$)-alkyl;
10. Aryl mit 6 - 12 C-Atomen;
11. ($C_7$-$C_{20}$)-Aralkyl;
12. Phthalidyl;
13. einen Rest der Formel

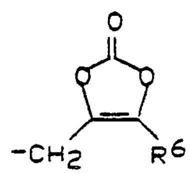

worin $R^6$ Wasserstoff, ($C_1$-$C_6$)-Alkyl oder Aryl mit 6 - 12 C-Atomen ist,
14. einen Rest der Formel

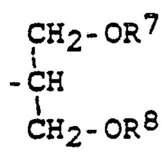

bedeuten, worin $R^7$ und $R^8$ gleich oder verschieden unabhängig voneinander Wasserstoff, einen gegebenenfalls substituierten, unverzweigten oder verzweigten, gesättigten oder ungesättigten Alkylrest mit 1 - 23 C-Atomen oder einen gegebenenfalls substituierten, unverzweigten oder verzweigten, gesättigten oder ungesättigten Acylrest mit 1 - 23 C-Atomen bedeuten,
wobei die unter d) 8., 9., 10., 11. und 12. genannten Reste wie unter b) 5. beschrieben im Arylteil substituiert wein können; und
wobei mindestens einer der Reste $R^2$ oder $R^3$ einen Rest der Formel

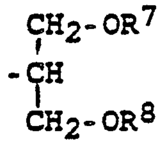

bedeutet, und

e) $R^4$ und $R^5$ zusammen mit den sie tragenden Atomen ein mono-, bi- oder tricyclisches heterocyclisches Ringsystem mit 3 bis 15 Ring-C-Atomen bilden;
sowie deren physiologisch verträgliche Salze mit Säuren und Basen.

Besonders bevorzugt sind Verbindungen der Formel I, in welchen
n = 1 oder 2 ist
R = Wasserstoff,
Alkyl mit 1 - 8 C-Atomen,
Alkenyl mit 2 - 6 C-Atomen, Cycloalkyl mit 3 - 9 C-Atomen,
Aryl mit 6 - 12 C-Atomen,
das durch ($C_1$-$C_4$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Alkanoylamino, Methylendioxy, Carboxy, Cyano und/oder Sulfamoyl, mono-, di- oder trisubstituiert sein kann,
Alkoxy mit 1 - 4 C-Atomen,
Aryloxy mit 6 - 12 C-Atomen,
das wie oben bei Aryl beschrieben substituiert sein kann,
mono- bzw. bicyclisches Heteroaryloxy mit 5 - 7 bzw. 8 - 10 Ringatomen, wovon 1 bis 2 Ringatome Schwefel-oder Sauerstoffatome und/oder 1 bis 4 Ringatome Stickstoff darstellen,
das wie oben bei Aryl beschrieben substituiert sein kann,
Amino-($C_1$-$C_4$)-alkyl,
($C_1$-$C_4$)-Alkanoylamino-($C_1$-$C_4$)alkyl,
($C_7$-$C_{13}$)-Aroylamino-($C_1$-$C_4$)-alkyl,
($C_1$-$C_4$)-Alkoxy-carbonylamino-($C_1$-$C_4$)-alkyl,
($C_6$-$C_{12}$)-Aryl-($C_1$-$C_4$)-alkoxycarbonylamino-($C_1$-$C_4$)-alkyl,
$C_6$-$C_{12}$)-Aryl-($C_1$-$C_4$)-alkylamino-($C_1$-$C_4$)-alkyl,
($C_{12}$-$C_4$)-alkylamino-($C_1$-$C_4$)-alkyl,
Di-($C_1$-$C_4$)-alkylamino-($C_1$-$C_4$)-alkyl,
Guanidino-($C_1$-$C_4$)-alkyl,
Imidazolyl, Indolyl,
($C_1$-$C_4$)-Alkylthio,
($C_1$-$C_4$)-Alkylthio-($C_1$-$C_4$)-alkyl,
($C_6$-$C_{12}$)-Arylthio- ($C_1$-$C_4$)-alkyl,
das im Arylteil wie oben bei Aryl beschrieben, substituiert sein kann,
($C_6$-$C_{12}$)-Aryl-($C_1$-$C_4$)-alkylthio,
das im Arylteil wie oben bei Aryl beschrieben substituiert sein kann,
Carboxy-($C_1$-$C_4$)-alkyl,
Carboxy, Carbamoyl,
Carbamoyl-($C_1$-$C_4$)-alkyl,
($C_1$-$C_4$)-Alkoxy-carbonyl-($C_1$-$C_4$)-alkyl,
($C_6$-$C_{12}$)-Aryloxy-($C_1$-$C_4$)-alkyl,
das im Arylteil wie oben bei Aryl beschrieben substituiert sein kann oder
($C_6$-$C_{12}$)-Aryl-($C_1$-$C_4$)-alkoxy,
das im Arylteil wie oben bei Aryl beschrieben substituiert sein kann,
R' Wasserstoff,
Alkyl mit 1 - 6 C-Atomen,
Alkenyl mit 2 - 6 C-Atomen,
Alkinyl mit 2 - 6 C-Atomen,
Cycloalkyl mit 3 - 9 C-Atomen,
Cycloalkenyl mit 5 - 9 C-Atomen,
($C_3$-$C_9$)-Cycloalkyl-($C_1$-$C_4$)-alkyl,

$(C_5-C_9)$-Cycloalkenyl-$(C_1-C_4)$-alkyl,
gegebenenfalls teilhydriertes Aryl mit 6 - 12 C-Atomen,
das wie oben bei R beschreiben substituiert sein kann,
$(C_6-C_{12})$-Aryl-$(C_1-C_4)$-alkyl oder $(C_7-C_{13})$-Aroyl-($C_1$ oder $C_2$)alkyl
die beide wie das vorstehende Aryl substituiert sein können
mono- bzw. bicyclisches, gegebenenfalls teilhydriertes Heteroaryl mit 5 - 7 bzw. 8 - 10 Ringatomen, wovon 1 bis 2 Ringatome Schwefel- oder Sauerstoffatome und/oder 1 bis 4 Ringatome Stickstoffatome darstellen,
das wie das vorstehende Aryl substituiert sein kann oder
die gegebenenfalls geschützte Seitenkette einer natürlichen vorkommenden α-Aminosäure $R^1$-$CH(NH_2)$-COOH bedeutet,
$R^2$ und $R^3$ gleich oder verschieden sind und
Wasserstoff,
Alkyl mit 1 - 6 C-Atomen,
Alkenyl mit 2 - 6 C-Atomen,
Di-$(C_1-C_4)$-alkylamino-$(C_1-C_4)$-alkyl,
$(C_1-C_5)$-Alkanoyloxy-$(C_1-C_4)$-alkyl,
$C_1-C_6)$-Alkoxy-carbonyloxy-$(C_1-C_4)$-alkyl,
$(C_7-C_{13})$-Aroyloxy-$(C_1-C_4)$-alkyl,
$(C_6-C_{12})$-Aryloxycarbonyloxy$(C_1-C_4)$-alkyl,
Aryl mit 6 - 12 C-Atomen,
$(C_6-C_{12})$-Aryl-$(C_1-C_4)$-alkyl,
$(C_3-C_9)$-Cycloalkyl,
$(C_3-C_9)$-Cycloalkyl-$(C_1-C_4)$-alkyl,
Phthalidyl,
einen Rest der Formel

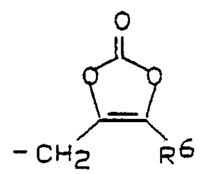

worin $R^6$ Wasserstoff, $(C_1-C_6)$-Alkyl oder Aryl mit 6 - 12 C-Atomen ist,
einen Rest der Formel

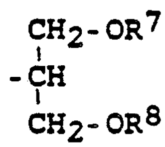

bedeuten, worin $R^7$ und $R^8$ gleich oder verschieden unabhängig voneinander Wasserstoff oder einen gegebenenfalls substituierten, unverzweigten oder verzweigten, gesättigten oder ungesättigten Acylrest mit 1 - 23 C-Atomen bedeuten, und wobei mindestens einer der Reste $R^2$ oder $R^3$ einen Rest der Formel

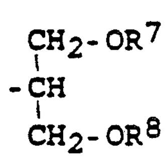

bedeutet, und
$R^4$ und $R^5$ die oben angegebene Bedeutung haben sowie deren physiologisch verträgliche Salze mit Säuren und Basen.

Ganz besonders bevorzugt sind Verbindungen der Formel I, in welchen
n = 1 oder 2 ist,

R $(C_1-C_6)$-Alkyl, $(C_2-C_6)$-Alkenyl, $(C_3-C_9)$-Cycloalkyl, Amino-$(C_1-C_4)$-alkyl, $(C_2-C_5)$-Acylamino-$(C_1-C_4)$-alkyl, $(C_7-C_{13})$-Aroylamino-$(C_1-C_4)$-alkyl, $(C_1-C_4)$-Alkoxycarbonylamino- $(C_1-C_4)$-alkyl, $(C_6-C_{12})$-Aryl-$(C_1-C_4)$-alkoxycarbonylamino-$(C_1-C_4)$-alkyl, $(C_6-C_{12})$-Aryl, das durch $(C_1-C_4)$-Alkyl, $(C_1-C_4)$-Alkoxy, Hydroxy, Halogen, Nitro, Amino, $(C_1-C_4)$-Alkylamino, Di-$(C_1-C_4)$-alkylamino und/oder Methylendioxy mono-, di-oder trisubstituiert sein kann, oder 3-Indolyl, insbesondere Methyl, Ethyl, Cyclohexyl, tert. Butoxycarbonylamino-$(C_1-C_4)$-alkyl, Benzyloxycarbonylamino-$(C_1-C_4)$-alkyl oder Phenyl, das durch Phenyl, $(C_1-C_2)$-Alkyl, $(C_1$ oder $C_2)$-Alkoxy, Hydroxy, Fluor, Chlor, Brom, Amino, $(C_1-C_4)$-Alkylamino, Di-$(C_1-C_4)$alkylamino, Nitro und/oder Methylendioxy mono- oder disubstituiert oder im Falle von Methoxy, trisubstituiert sein kann, bedeutet,

$R^1$ Wasserstoff oder $(C_1-C_6)$-Alkyl, das gegebenenfalls durch Amino, $(C_1-C_6)$Acylamino oder Benzoylamino substituiert sein kann, $(C_2-C_6)$-Alkenyl, $(C_3-C_9)$-Cycloalkyl, $(C_5-C_9)$-Cycloalkenyl, $(C_3-C_7)$-Cycloalkyl-$(C_1-C_4)$-alkyl, $(C_6-C_{12})$-Aryl oder teilhydriertes Aryl, das jeweils durch $(C_1-C_4)$-Alkyl, $(C_1$ oder $C_2)$-Alkoxy oder Halogen substituiert sein kann, $(C_6-C_{12})$-Aryl-$(C_1$ bis $C_4)$-alkyl oder $(C_7-C_{13})$-Aroyl-$(C_1-C_2)$alkyl, die beide wie vorstehend definiert im Arylrest substituiert sein können, ein mono- bzw. bicyclischer Heterocyclen-Rest mit 5 bis 7 bzw. 8 bis 10 Ringatomen, woven 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure, insbesondere aber Wasserstoff, $(C_1-C_3)$-Alkyl, $(C_2$ oder $C_3)$-Alkenyl die gegebenenfalls geschützte Seitenkette von Lysin, Phenylalanin oder Tyrosin, Benzyl, 4-Methoxybenzyl, 4-Ethoxybenzyl, Phenethyl, 4-Amino-butyl oder Benzoylmethyl bedeutet,

$R^2$ und $R^3$ gleiche oder verschiedene Reste Wasserstoff, $(C_1-C_6)$-ALkyl, $(C_2-C_6)$-Alkenyl, $(C_6-C_{12})$-Aryl-$(C_1-C_4)$-alkyl, $(C_1-C_5)$-Alkanoyloxy-$(C_1-C_4)$-alkyl, $(C_1-C_6)$-Alkoxycarbonyloxy-$(C_1-C_4)$-alkyl, $(C_7-C_{13})$-Aroyloxy-$(C_1-C_4)$-alkyl, $(C_6-C_{12})$-Aryloxycarbonyloxy-$(C_1-C_4)$-alkyl, Phthalidyl, einen Rest der Formel

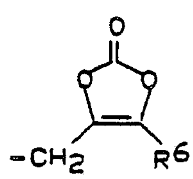

worin $R^6$ Wasserstoff, $(C_1-C_6)$-Alkyl oder Aryl mit 6 -12 C-Atomen ist, oder einen Rest der Formel

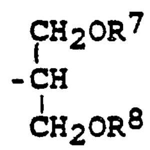

worin $R^7$ und $R^8$ Wasserstoff oder einen gesättigten oder ungesättigten Acylrest mit 8 - 23 C-Atomen bedeuten und wobei mindestens einer der Reste $R^2$ oder $R^3$ einen Rest der Formel

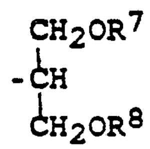

bedeutet und

$R^4$ und $R^5$ die oben angegebene Bedeutung haben sowie deren physiologisch verträgliche Salze mit Säure und Basen.

Beispiele ganz besonders bevorzugter Verbindungen sind:

2-[N-(1S-Ethoxycarbonyl-3-phenylpropyl)-S-alanyl]-1,2,3,4-tetrahydroisochinolin-3S-carbonsäure-(1,3-dihydroxy-2-propyl)ester;

2-[N-(1S-Ethoxycarbonyl-3-phenylpropyl)-S-alanyl]-1,2,3,4-tetrahydroisochinolin-3S-carbonsäure-[1,3-di-(hexadecanoyloxy)-2-propyl]ester;

2-[N-(1S-Ethoxycarbonyl-3-phenylpropyl)-S-alanyl]-1,2,3,4-tetrahydroisochinolin-3S-carbonsäure-[1,2-di-(9Z, 12Z-octadecadienoyl)-2-propyl]ester;

2-[N-(1S-Carboxy-3-phenylpropyl)-S-alanyl]1,2,3,4-tetrahydroisochinolin-3S-carbonsäure-[1,3-di-(tetradecanoyloxy)-2-propyl]ester;

2-[N-[1S-[1,3-Di-(octadecanoyloxy)-2-propyloxycarbonyl]-3-phenylpropyl)-S-alanyl]-1,2,3,4-tetrahydroisochinolin-3S-carbonsäure;
2-[N-[1S-[1,3-Di(9Z,12Z,15Z-octadecatrienoyloxy)-2-propyloxycarbonyl]-3-phenylpropyl]-S-alanyl]-1,2,3,4-tetrahydroisochinolin-3S-carbonsäure-n-octylester;
2-[N-[1S-(1,3-Dihydroxy-2-propyloxycarbonyl)-3-phenylpropyl]-S-alanyl]-1,2,3,4-tetrahydroisochinolin-3S-carbonsäure-(1,3-dihydroxy-2-propyl)ester;
1-[N-(1S-Ethoxycarbonyl-3-phenylpropyl)-S-alanyl]-(2S,3aR,7aS)-octahydro[1H]indol-2-carbonsäure-[1,3-di-(octadecanoyloxy)-2-propyl]ester;
1-[N-(1S-Octyloxycarbonyl-3-phenylpropyl)-S-alanyl]-(2S,3aR,7aS)-octahydro[1H]indol-2-carbonsäure-[1,3-di(5Z,8Z,11Z,14Z-eicosatetraenoyloxy)-2-propyl]ester;
1-[N-(1S-Menthyloxycarbonyl-3-phenylpropyl)-S-alanyl]-(2S,3aR,7aS)-octahydro[1H]indol-2-carbonsäure-[1,3-di(hexadecanoyloxy)-2-propyl]ester;
1-[N-(1S-[1,3-Di(tetradecanoyloxy)-2-propyloxycarbonyl]-3-phenylpropyl]-S-alanyl]-(2S,3aR,7aS)-octahydro-[1H]indol-2-carbonsäure-[1,3-di(tetradecanoyloxy)-2-propyl]ester;
1-[N-[1S-[1,3-Di(9Z,12Z-octadecadienoyloxy)-2-propyloxycarbonyl]-3-phenylpropyl]-S-alanyl]-(2S,3aR,7aS)-octahydro[1H]indol-2-carbonsäure-(5-methyl-1,3-dioxolen-2-on-4-yl)methylester;
1-[N-[1S-(1,3-Dihydroxy-2-propyloxycarbonyl)-3-phenylpropyl]-S-alanyl]-(2S,3aR,7aS)-octahydro[1H]indcl-2-carbonsäure-(5-nonyl)ester;
1-[N-(1S-Carboxy-3-phenylpropyl)-S-alanyl]-(2S,3aS,7aS)-octahydro[1H]indol-2-carbonsäure-[1,3-di-(hexadecanoyloxy)-2-propyl]ester;
1-[N-(1S-Ethoxycarbonyl-3-phenylpropyl)-S-alanyl]-(2S,3aS,7aS)(-octahydro[1H]indol-2-carbonsäure-[1,3-di9Z,12Z,14Z-octadecatrienoyloxy)-2-propyl]ester;
1-[N-(1S-Ethoxycarbonyl-3-phenylpropyl)-S-alanyl]-(2S,3aS,7aS)-octahydro[1H]indol-2-carbonsäure-[1,3-dihydroxy-2-propyl)ester;
1-[N-[1S-[1,3-Di(hexadecanoyloxy)-2-propyloxycarbonyl]-3-phenylpropyl]-S-alanyl]-(2S, 3aS,7aS)-octahydro-[1H]indol-2-carbonsäure-[1,3-di(hexadecanoyloxy)-2-propyl]ester;
1-[N-1S-[1,3-Di(octadecanoyloxy)-2-propyloxy-carbonyl]-3-phenylpropyl]-S-alanyl]-(2S,3aS,7aS)-octahydro-[1H]indol-2-carbonsäure;
1-[N[1S-[1,3-Di(9Z-octadecenoyloxy)-2-propyloxycarbonyl]-3-phenylpropyl]-S-alanyl]-(2S,3aS,7aS)-octahydro-[1H]indol-2-carbonsäurebenzhydrylester;
2-[N-(1S-Ethoxycarbonyl-3-phenylpropyl)-S-alanyl]-(1S,3S,5S)-2-azabicyclo[3.3.0]octan-3-carbonsäure-(1,3-dihydroxy-2-propyl)ester;
2-(N-(1S-Ethoxycarbonyl-3-phenylpropyl)-S-alanyl]-(1S,3S,5S)-2-azabicyclo[3.3.0]octan-3-carbonsäure-(1,3-dipivaloyloxy-2-propyl)ester;
2-[N-[1S-Ethoxycarbonyl-3-phenylpropyl)-S-alanyl]-(1S,3S,5S)-2-azabicyclo[3.3.0]octan-3-carbonsäure-[1,3-di(decanoyloxy)-2-propyl]ester;
2-[N-(1S-Ethoxycarbonyl-3-phenylpropyl)-S-alanyl]-(1S,3S,5S)-2-azabicyclo[3.3.0]octan-3-carbonsäure-[1,3,di(hexadecanoxyloxy)-2-propyl]ester;
2-[N-(1S-Ethoxycarbonyl-3-phenylpropyl)-S-alanyl]-(1S,3S,5S)-2-azabicyclo[3.3.0]octan-3-carbonsäure-[1.3-di(octadecanoyloxy)-2-propyl]ester;
2-[N-(1S-Carboxy-3-phenylpropyl)-S-alanyl]-(1S,3S,5S)-2-azabicyclo[3.3.0]octan-3-carbonsäure-[1,3-di-(5Z,8Z,11Z,14Z-eicosatetraenoyloxy)-2-propyl]ester;
2-[N-[1S-[1,3-Di(hexadecanoyloxy)-2-propyloxycarbonyl]-3-phenylpropyl]-S-alanyl]-(1S,3S,5S)-2-azabicyclo-[3.3.0]octan-3-carbonsäureoctylester;
2-[N-1S-[1,3-Di(octadecanoyloxy)-2-propyloxycarbonyl]-3-phenylpropyl]-S-alanyl]-(1S,3S,5S)-2-azabicyclo-[3.3.0]octan-3-carbonsäure;
2-[N-[1S-(1,3-Dihydroxy-2-propyloxycarbonyl)-3-phenylpropyl]-S-alanyl]-(1S,3S,5S)-2-azabicyclo[3.3.0]octan-3-carbonsäure-(1,3-dihydroxy-2-propyl)ester;
2-[N-[1S-[1,3-Di(9Z,12Z-octadecadienoyloxy-2-propyloxycarbonyl]-3-phenylpropyl]-S-alanyl]-(1S,3S,5S)-2-azabicyclo[3.3.0]-octan-3-carbonsäuredecylester;
1-[N-(1S-Ethoxycarbonyl-3-phenylpropyl]-S-alanyl]-3′S-spiro[(bicyclo[2.2.2]octan)-2,3′-pyrrolidin]-5′S-yl-carbonsäure[1,3-dihexadecanoyloxy-2-propyl]ester;
1-[N-(1S-Octyloxycarbonyl-3-phenylpropyl]-S-alanyl]-3′S-spiro[(bicyclo[2.2.0]octan)-2,3′-pyrrolidin]-5′S-yl-carbonsäure-(1-octadecanoyloxy-3-octadecanyloxy-2-propyl)-ester;
1-[N(1S-Carboxy-3-phenylpropyl)-S-alanyl]-3′S-spiro[2.2.2]octan)-2,3′-pyrrolidin]-5′S-yl-carbonsäure-[1,3-di-(9Z,12Z,15Z-octadecatrienoyloxy)-2-propyl]ester;
1-[N-(1S-Menthyloxycarbonyl-3-phenylpropyl]-S-alanyl]-3′S-spiro[(bicyclo[2.2.2.]octan)-2,3′-pyrrolidin]-5′S-yl-carbonsäure-(1,3-dihydroxy-2-propyl]ester;
1-[N-[1S-[1,3-Di(tetradecanoyloxy)-2-propyloxycarbonyl]-3-phenylpropyl]-S-alanyl]3′S-spiro[(bicyclo[2.2.2]-

octan)-2,3'-pyrrolidin]-5'S-yl-carbonsäure-[1,3-di(tetradecanoyloxy)-2-propyl]ester;
1-[N-[1S-[1,3-Di(octadecanoyloxy)-2-propyloxycarbonyl]-3-phenylpropyl]-S-alanyl]3'S-spiro[(bicyclo[2.2.2]-octan)-2,3'-pyrrolidin]-5'S-yl;-carbonsäuredecylester;
1-[N-[1S-(1,3-Dihydroxy-2-propyloxycarbonyl)-3-phenylpropyl]-S-alanyl]-3'S-spiro[(bicyclo[2.2.2]octan)-2,3'-pyrrolidin]-5'S-yl-carbonsäure-(5-nonyl)ester;
2-[N-(1S-Ethoxycarbonyl-3-phenylpropyl]-S-alanyl]-(1S,3S,5S)-2-azabicyclo[3.3.0]-7-octen-3-carbonsäure-[1,3-di-(hexadecanoyloxy)-2-propyl]ester;
2-[N-(1S-Carboxy-3-phenylpropyl]-S-alanyl]-(1S,3S,5S)-2-azabicyclo[3.3.0]-7-octen-3-carbonsäure(1-hexadecanyloxy)-3-tetradecanoyloxy-2-propyl)ester;
2-[N-(1S-Nonyloxycarbonyl-3-phenylpropyl]-S-alanyl]-(1S,3S,5S)-2-azabicyclo[3.3.0]-7-octen-3-carbonsäure-(1,3-di(9Z-octadecenoyloxy)-2-propyl)ester;
2-[N-(1S-[1,3-Di(hexadecanoyloxy]-2-propyloxycarbonyl]-3-phenylpropyl]-S-alanyl]-(1S,3S,5S)-2-azabicyclo-[3.3.0]-7-octen-3-carbonsäure-[1,3-di-(hexadecanoyloxy)-2-propyl]ester;
2-[N-[1S-(1,3-Dihydroxy-2-propyloxycarbonyl)-3-phenylpropyl]-S-alanyl]-(1S,3S,5S)-2-azabicyclo[3.3.0]-7-octen-3-carbonsäure;
2-[N-[1S-[1,3-Di(octadecanoyloxy)-2-propyloxycarbonyl]-3-phenylpropyl]-S-alanyl]-(1S,3S,5S)-2-azabicyclo-[3.3.0]-7-octen-3-carbonsäurebenzhydrylester;
1-[N-(1S-Ethoxycarbonyl-3-phenylpropyl)-S-alanyl]-pyrrolidin-2S-carbonsäure-[1,3-di(hexadecanoyloxy)-2-propyl]ester;
1[N-(1S-Carboxy-3-phenylpropyl-S-alanyl]-pyrrolidin-2S-carbonsäure-[1,3-di(5Z,8Z,11Z,14Z-eicosatetraenoyloxy)-2-propyl]ester;
1-[N-(1S-Octyloxycarbonyl-3-phenylpropyl)-S-lysyl]-pyrrolidin-2S-carbonsäure-[1,3-dihydroxy-2-propyl]ester;
1-[N-[1S-[1,3-Di-(eicosanoyloxy)-2-propyloxycarbonyl]-3-phenylpropyl]-S-alanyl]-pyrrolidin-S-carbonsäure-[1,3-di(eicosanoyloxy)-2-propyl]ester;
1-[N-1S-[1,3-Di(tetradecanoyloxy)-2-propyloxycarbonyl]-3-phenylpropyl]-S-lysyl-pyrrolidin-2S-carbonsäure-(5-nonyl)ester;
1-[N-[1S-[1,3-Di(hexadecanyloxy-2-propyloxycarbonyl]-3-phenylpropyl]-S-lysyl]-pyrrolidin-2S-carbonsäureundecylester.

Als Salze der Verbindungen der Formel I kommen, je nach saurer oder basischer Natur dieser Verbindungen, Alkali-oder Erdalkalisalze oder Salze mit physiologisch verträglichen Aminen oder Salze mit anorganischen oder organischen Säuren wie z. B. HCl, HBr, $H_2SO_4$, Maleinsäure, Fumarsäure, Weinsäure, Citronensäure in Frage.

Die Erfindung betrifft ferner ein Verfahren zur Herstellung von Verbindungen der Formel I, dadurch gekennzeichnet, daß man

a) eine Verbindung der Formel II

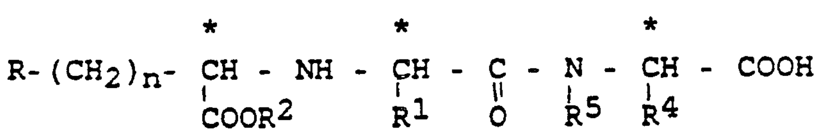

(II)

in der R, $R^1$, $R^2$, $R^4$, $R^5$ und n die gleiche Bedeutung wie in Formel I haben, mit einer Verbindung der Formel IIIa/b

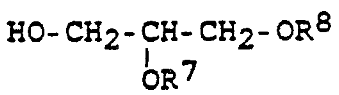

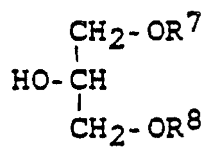

(IIIa) (IIIb)

in der $R^7$ und $R^8$ die gleiche Bedeutung wie in Formel I besitzen, unter Anwendung dem Fachmann vertrauter Veresterungsmethoden (siehe z. B. Buchler, Pearson, Survey of Organic Synthesis, Vol. 1, New York 1970, S. 802 - 825; Houben-Weyl, Methoden der Organischen Chemie, Band E5, 1985, S. 656 - 773), z.B. unter saurer Katalyse oder nach Aktivierung der Carbonsäurefunktion von II bzw. der Hydroxyfunktion

von IIIa/b, insbesondere unter den Bedingungen einer Mitsunobu-Reaktion, in einem geeignetem Lösungsmittel bei einer Temperatur bis zum Siedepunkt des Reaktionsgemisches umsetzt
oder daß man

b) eine Verbindung der Formel II, in der R, $R^1$, $R^2$, $R^4$, $R^5$ und n die gleiche Bedeutung wie in Formel I haben, mit einer Verbindung der Formel IVa/b

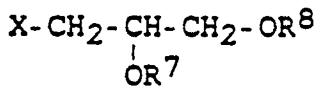 (IVa) 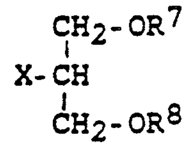 (IVb)

in der $R^7$ und $R^8$ die gleiche Bedeutung wie in Formel I haben, und
in der X eine Abgangsgruppe, die nucleophil verdrängt werden kann, insbesondere ein Cl, Br, J-Atom oder einen Sulfonsäurerest, bedeutet, unter Bedingungen einer nucleophilen Substitution, vorzugsweise in einem polaren organischen Lösungsmittel, wie einem Alkohol, vorzugsweise Methanol, Ethanol, Propanol oder Isopropanol oder einem niederen Keton, vorzugsweise Aceton, Methylethylketon oder Methylisobutylketon oder in Acetonitril, Dimethylformamid, Dimethylsulfoxid oder Sulfolan oder einem Kohlenwasserstoff, vorzugsweise Toluol, mit oder ohne Gegenwart einer Hilfsbase zum Abfangen der sich bildenden Säure, vorzugsweise in Gegenwart von Kaliumhydrogencarbonat, Kaliumcarbonat, Natriumhydrogencarbonat, Natriumcarbonat, Triethylamin, Pyridin, 1,5-Diazabicyclo[5,4,0]undec-5-en oder 1,5-Diazabicyclo[4,3,0]non-5-en, sowie mit oder ohne Gegenwart eines Alkalihalogenids, vorzugsweise Natriumjodid oder Kaliumjodid, bei einer Temperatur zwischen -50 und +100 °C, vorzugsweise zwischen -20 und +60 °C, umsetzt, oder daß man

c) eine Verbindung der Formel V

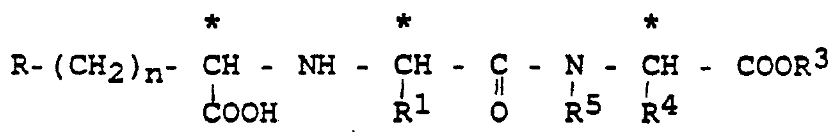 (V)

in der R, $R^1$, $R^3$, $R^4$, $R^5$ und n die gleiche Bedeutung wie in Formel I haben, mit einer Verbindung der Formel IIIa/b wie unter der Verfahrensvariante a) beschrieben, umsetzt, oder daß man,

d) eine Verbindung der Formel V, in der R $R^1$, $R^3$, $R^4$, $R^5$ und n die gleiche Bedeutung wie in Formel I haben, mit einer Verbindung der Formel IVa/b, wie unter der Verfahrensvariante b) beschrieben, umsetzt, oder daß man

e) eine Verbindung der Formel VI

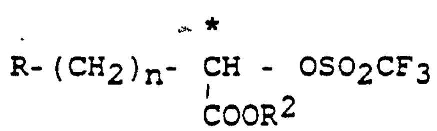 (VI)

worin R, $R^2$ und n die gleiche Bedeutung wie in Formel I haben, mit einer Verbindung der Formel VII

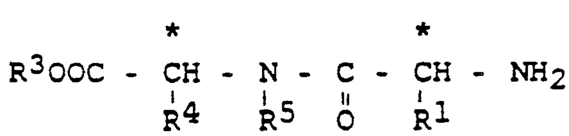 (VII)

worin $R^1$, $R^3$, $R^4$ und $R^5$ die gleiche Bedeutung wie in Formel I haben, z.B. analog der in US-Patent 4525301 beschriebenen Verfahrensweise in einem geeigneten Lösungsmittel bei einer Temperatur bis zum Siedepunkt des Reaktionsgemisches umsetzt,
oder daß man

Verbindungen der Formel VI, in welcher $R^2$ den Rest

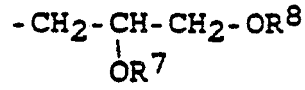 oder 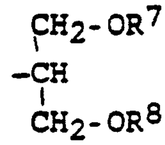

bedeutet, werden aus Verbindungen der Formel XII

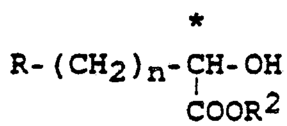 (XII)

worin R, $R^2$ und n die gleiche Bedeutung wie in Formel I besitzen, durch Überführung der Hydroxylfunktion in die $-OSO_2CF_3$-Gruppe nach gängigen Verfahren erhalten.

Verbindungen der Formel VII sind Dipeptide, die aus den einzelnen Aminosäurekomponenten nach an sich bekannten Methoden der Peptidchemie (siehe z.B. Houben-Weyl, Methoden der Organischen Chemie, Bd. XV, Teil II, S. 1-364) aufgebaut werden können.

Verbindungen der Formel VIII, in welchen $R^2$ den Rest

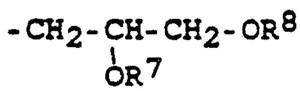 oder 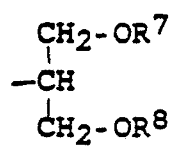

bedeutet, werden aus Verbindungen der Formel XIII

HOOC-CH=CH-CO-R (XIII)

in welcher R die gleiche Bedeutung wie in Formel I hat, durch Umsetzung mit Verbindungen der Formel IIIa/b unter Veresterungsbedingungen, wie unter der Verfahrensvarinate a) beschrieben, oder durch Umsetzung mit Verbindungen der Formel IVa/b unter den Bedingungen einer nucleophilen Substitution, wie unter der Verfahrensvariante b) beschrieben, erhalten.

Verbindungen der Formel IX, in welchen $R^2$ den Rest

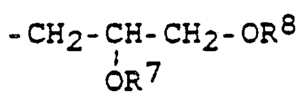 oder 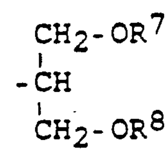

bedeutet, werden aus Verbindungen der Formel XIV

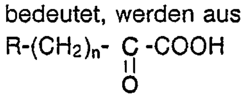 (XIV)

worin R und n die gleiche Bedeutung wie in Formel I besitzen, durch Umsetzung mit verbindungen der Formel IIIa/b unter Veresterungsbedingungen, wie unter der Verfahrensvariante a) angegeben, oder durch Umsetzung mit Verbindungen der Formel IVa/b unter den Bedingungen einer nucleophilen Substitution, wie unter der Verfahrensvariante b) beschrieben, erhalten.

Verbindungen der Formel X lassen sich herstellen aus Verbindungen der Formel II durch Umsetzung mit 1,2- bzw. 1,3-geschützten Glycerinderivaten, wie sie zahlreich bekannt sind, unter Veresterungsbedingungen, wie sie unter der Verfahrensvariante a) beschrieben sind, bzw. unter Bedingungen einer nucleophilen Substitution, wie sie unter der Verfahrensvariante b) beschrieben sind. Auf analogem Wege werden Verbindungen der Formel XI aus Verbindungen der Formel III erhalten.

Verbindungen der Formel XII erhält man z.B. aus Verbindungen der Formel XV

$$R-(CH_2)_n-\overset{*}{\underset{COOH}{CH}}-OH \qquad (XV)$$

durch alkylierende Veresterung der Carboxylgruppe, anschließenden Schutz der Hydroxyfunktion, z.B. mit einer hydrogenolytisch zu entfernenden Schutzgruppe, Verseifung des Esters, anschließende Reaktion mit einer Verbindung der Formel IIIa/b unter Veresterungsbedingungen, wie sie unter der Verfahrensvariante a) beschrieben sind, oder mit einer Verbindung der Formel IVa/b unter den Bedingungen einer nucleophilen Substitution, wie sie unter der Verfahrensvariante b) beschrieben sind, gefolgt von einer Abspaltung der Hydroxy-Schutzgruppe, oder durch eine äquivalente Reaktionsfolge.

Verbindungen der Formeln XIII, XIV, und XV sind bekannt.

Die Verbindungen der Formel I sind Glycerinester von Inhibitoren des Angiotensin-Converting-Enzyms (ACE-Hemmer). Triglyceride speziell von mittel- und langkettigen Fettsäuren besitzen einen speziellen Resorptionsmechanismus: Nach der micellaren Aufnahme in die Enterocyten des Dünndarms werden sie als Bestandteile der Chylomikronen nicht ins venöse Blut, sondern in die Lymphe entlassen. Von dort gelangen sie unter Umgehung der Leber ins Blut (siehe z.B.: L. Naupert et al., Klin. Wochenschrift 48, 449 (1970)).

Die Verbindungen der Formel I weisen gegenüber den bekannten ACE-Hemmern eine geänderte Pharmakokinetik und eine geänderte Gewebsverteilung auf, die sich bei einigen Vertretern in einer Zunahme der zentralen und einer relativen Abnahme der peripheren Wirkungen äußert. Unter physiologischen Bedingungen werden sie zumindest teilweise in die ACE-Hemmer gespalten.

Die Verbindungen der Formel I und deren Salze besitzen lang andauernde, intensive blutdrucksenkende Wirkung. Sie können zur Bekämpfung des Bluthochdrucks verschiedener Genese eingesetzt werden. Auch ihre Kombination mit anderen blutdrucksenkenden, gefäßerweiternden oder diuretisch wirksamen Verbindungen ist möglich. Typische Vertreter dieser Wirkklassen sind z.B. in Erhardt-Ruschig, Arzneimittel, 2. Auflage, Weinheim, 1972, beschrieben. Die Anwendung kann intravenös, subcutan oder peroral erfolgen.

Die Dosierung bei oraler Gabe liegt bei 1-1000 mg, vorzugsweise bei 1-400 mg je Einzeldosis bei einem normalgewichtigen erwachsenen Patienten: dies entspricht etwa 15-1300 µg/kg/Tag, vorzugsweise 15-5000 µg/kg/Tag. Sie kann in schweren Fällen auch erhöht werden, da toxische Eigenschaften bisher nicht beobachtet wurden. Auch eine Herabsetzung der Dosis ist möglich und vor allem dann angebracht, wenn gleichzeitig Diuretika verabreicht werden.

Die erfindungsgemäßen Verbindungen der Formel I sind aufgrund ihrer pharmakologischen Eigenschaften neben der Behandlung des Blutdruckes auch für die Behandlung cognitiver Dysfunktionen unterschiedlicher Genese, wie sie z.B. bei der Alzheimer'schen Krankheit oder der senilen Demenz auftreten, geeignet. Die nootrope Wirkung der erfindungsgemäßen Verbindungen wurde an Mäusen, die ein Körpergewicht von 20 - 25 g besaßen, im inhibitory (passive) avoidance test (step-through-Modell) geprüft. Eine modifizierte Form der von J. KOPP, Z. BODANECKY und M.E. JARVIK beschriebenen Testmethode wurde von J. BURES, O. BURESOVA und J. HUSTON in "Techniques and Basic Experiments for the Study of Brain and Behavior", Elsevier Scientific Publishers, Amsterdam (1983) beschrieben.

Entsprechend diesen Literaturangaben wird eine Substanz dann als nootrop wirksam bezeichnet, wenn sie bei den Versuchstieren die mittels eines elektroconvulsiven Schocks erzeugte Amnesie oder die mittels Scopolamin induzierte Amnesie aufzuheben vermag.

Die Versuche wurden nach modifizierten Testmethoden durchgeführt. Als Vergleichsverbindung diente das bekannte Nootropikum 2-Oxo-1-pyrrolidinylessigsäureamid (Piracetam). Die deutliche Überlegenheit der erfindungsgemäßen Verbindungen über die Vergleichssubstanz zeigte sich darin, daß die Scopolamininduzierte Amnesie im inhibitory avoidance-Test sich mit einer MED (minimal effective Dosis) von 0,03 - 30 mg/kg p.o. aufheben läßt. Die Vergleichssubstanz hat eine MED von ca. 500-1000 mg/kg p.o.

Die Erfindung betrifft daher weiter die Anwendung der erfindungsgemäßen Verbindungen bei der Behandlung und Prophylaxe cognitiver Dysfunktionen.

Die Erfindung umfaßt weiterhin die genannten Verbindungen enthaltende Arzneimittel, Verfahren zu deren Herstellung sowie die Verwendung der erfindungsgemäßen Verbindungen bei der Herstellung von Arzneimitteln, die zur Behandlung und Prophylaxe der vorstehend genannten Krankheiten eingesetzt werden.

In Ausübung der erfindungsgemäßen Methode können die oben beschriebenen Verbindungen der Formel I an Säugern wie Affen, Hunden, Katzen, Ratten, Menschen etc. angewendet werden.

Die Arzneimittel werden nach an sich bekannten, dem Fachmann geläufigen Verfahren hergestellt. Als

Arzneimittel werden die erfindungsgemäßen pharmakologisch wirksamen Verbindungen (= Wirkstoff) entweder als solche oder vorzugsweise in Kombination mit geeigneten pharmazeutischen Hilfsstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 %, beträgt.

Welche Hilfsstoffe für die gewünschte Arzneimittelformulierung geeignet sind, ist dem Fachmann aufgrund seines Fachwissens geläufig. Neben Lösungsmitteln, Gelbildnern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Die Wirkstoffe können beispielsweise oral, rektal oder parenteral (z.B. intravenös oder subcutan) appliziert werden, wobei die orale Applikation bevorzugt ist.

Für eine orale Anwendungsform werden die aktiven Verbindungen mit den dafür geeigneten Zusatzstoffen wie Trägerstoffen, Stabilisatoren oder inerten Verdünnungsmitteln vermischt und durch die üblichen Methoden in geeignete Darreichungsform gebracht wie Tabletten, Dragees, Steckkapseln, wässrige, alkoholische oder ölige Suspensionen oder wässrige, alkoholische oder ölige Lösungen. Als inerte Träger können z.B. Gummi arabicum, Magnesia, Magnesiumcarbonat, Milchzucker, Glucose oder Stärke, insbesondere Maisstärke verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht wie Sonnenblumenöl oder Lebertran.

Zur subcutanen oder intravenösen Applikation werden die aktiven Verbindungen oder deren physiologisch verträglichen Salze, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage Wasser, physiologische Kochsalzlösung oder Alkohole, z.B. Ethanol, Propanol, Glycerin, daneben auch Zuckerlösungen wie Glucose- oder Mannitlösungen oder auch eine Mischung aus den verschiedenen genannten Lösungsmitteln.

Die nachfolgenden Beispiele sollen die erfindungsgemäßen Verbindungen und Verfahren erläutern, ohne die Erfindung auf die hier stellvertretend genannten Substanzen zu beschränken.

Beispiel 1:

2-[N-(1S-Ethoxycarbonyl-3-phenylpropyl]-S-alanyl]-(1S,3S,5S)-2-azabicyclo[3.3.0]-octan-3-carbonsäure-[(1,3-di(hexadecanoyloxy)-2-propyl]ester

2,75 g (10,5 mmol) Triphenylphosphin und 3,98 g (7,0 mmol) 1,3-Di(hexadecanoyloxy)-2-propanol werden in 140 ml absolutem Tetrahydrofuran gelöst und bei 0° C eine Lösung von 1,83 g (10,5 mmol) Azodicarbonsäurediethylester in 15 ml absolutem Tetrahydrofuran zugetropft. Nach 15 Minuten bei 0° C wird eine Lösung von 2,92 g (7,0 mmol) 2-[N-(1S-Ethoxycarbonyl-3-phenylpropyl)-S-alanyl]-(1S,3S,5S)-2-azabicyclo[3.3.0]octan-3-carbonsäure (Ramipril) in 35 ml absolutem Tetrahydrofuran hinzugetropft, eine Stunde bei 0° C und über Nacht bei Raumtemperatur nachgerührt. Das Reaktionsgemisch wird eingeengt, das Rohprodukt in 200 ml Ether gelöst, mit 3 x 100 ml 2 N Natronlauge und 1 x 100 ml Wasser extrahiert, über Magnesiumsulfat getrocknet, eingeeengt und durch Chromatographie an 1100 g Kieselgel (Laufmittel Methylenchlorid/Essigester 9:1, 8:2) von Verunreinigungen befreit. Man erhält 6,11 g (90%) farbloses wachsartiges Produkt.

$[\alpha]_D^{25}$ = -8,2° (c = 1, Methanol).

Unter Verwendung geeigneter Ausgangsmaterialien werden die nachstehenden Verbindungen analog der in Beispiel 1 angegebenen Vorschriften hergestellt.

**Beispiel 2:**

2-[N-(1S-Ethoxycarbonyl-3-phenylpropyl]-S-alanyl]-(1S,3S,5S)-2-azabicyclo[3.3.0]octan-3-carbonsäure-(1,3-di(decanoyloxy)-2-propyl)ester;

$[\alpha]_D^{25}$ = -11,5° (c = 1, Methanol).

**Beispiel 3:**

2-[N-(1S-Ethoxycarbonyl-3-phenylpropyl]-S-alanyl]-(1S,3S,5S)-2-azabicyclo[3.3.0]octan-3-carbonsäure-(1,3-di(decanoyloxy)-2-propyl)ester;

$[\alpha]_D^{25}$ = -6,8° (c = 1, Methanol).

**Beispiel 4:**

2-[N-(1S-Ethoxycarbonyl-3-phenylpropyl]-S-alanyl]-(1S,3S,5S)-2-azabicyclo[3.3.0]octan-3-carbonsäure-(1,3-di(dipivaloyloxy-2-propyl)ester;

$[\alpha]_D^{25}$ = -17,5° (c = 1,22, Methanol).

**Beispiel 5:**

2-[N-(1S-Ethoxycarbonyl-3-phenylpropyl)-S-alanyl]-(1S,3S,5S)-2-azabicyclo[3.3.0]octan-3-carbonsäure-(1,3-di(hydroxy)-2-propyl)ester;

0,48 g (0,98 mmol) 2-[N-(1S-Ethoxycarbonyl-3-phenylpropyl)-S-alanyl]-(1S,3S,5S)-2-azabicyclo[3.3.0]-octan-3-carbonsäure-(2-Phenyl-1,3-dioxan-5-yl)-ester und 1,5 g p-Toluolsulfonsäure werden in 50 ml Tetrahydrofuran 2 Std. am Rückfluß gekocht. Die Reaktionslösung wird eingeengt, der Rückstand in Essigester aufgenommen, mehrmals mit gesättigter Natriumbicarbonatlösung gewaschen, getrocknet, eingeengt und durch Säulenchromatographie an Kieselgel (Laufmittel Toluol/Ethanol 98:2, 95:5, 9:1) gereinigt. Man erhält 0,056 g des Produktes.
MS (FAB): 491 (M+1).

Der als Ausgangsmaterial verwendete 2-[N-(1S-Ethoxycarbonyl-3-phenylpropyl)-S-alanyl]-(1S,3S,5S)-2-azabicyclo[3.3.0]-octan-3-carbonsäure-(2-phenyl-1,3-dioxan-5-yl)ester wird analog Beispiel 1 aus 5-Hydroxy-2-phenyl-1,3-dioxan und 2-[N-)1S-Ethoxycarbonyl-3-phenylpropyl)-S-alanyl]-(1S,3S,5S)-2-azabicyclo[3.3.0]-octan-3-carbonsäure (Ramipril) erhalten.

Unter Verwendung geeigneter Ausgangsmaterialien können die nachstehenden Verbindungen analog in Beispiel 1 angegebenen Vorschrift hergestellt werden.
2-[N-(1S-Ethoxycarbonyl-3-phenylpropyl)-S-alanyl]-1,2,3,4-tetrahydroisochinolin-3S-carbonsäure-[1,3-di-(hexadecanoyloxy)-2-propyl]ester;
2-[N-(1S-Ethoxycarbonyl-3-phenylpropyl)-S-alanyl]-1,2,3,4-tetrahydroisochinolin-3S-carbonsäure-[1,3-di-(9Z,12Z-octadecadienoyl)-2-propyl]ester;
2-[N-(1S-Carbonyl-3-phenylpropyl)-S-alanyl]-1,2,3,4-tetrahydroisochinolin-3S-carbonsäure-[1,3-di-(tetradecanoyloxy)-2-propyl]ester;
2-[N-[1S[1,3-Di-(octadecanoyloxy)-2-propyloxycarbonyl]-3-phenylpropyl)-S-alanyl]-1,2,3,4-tetrahydroisochinolin-3S-carbonsäure;
2-[N-[1S-[1,3-Di-(9Z,12Z,15Z-octadecatrienoyloxy)-2-propyloxycarbonyl]-3-phenylpropyl]-S-alanyl]-1,2,3,4-tetrahydroisochinolin-3S-carbonsäure-n-octylester;
1-[N-(1S-Ethoxycarbonyl-3-phenylpropyl)-S-alanyl]-(2S,3aR,7aS)-octahydro[1H]indol-2-carbonsäure-[1,3-di-(octadecanoyloxy)-2-propyl]ester;
1-[N-(1S-Octyloxycarbonyl-3-phenylpropyl)-S-alanyl]-(2S,3aR,7aS)-octahydro[1H]indol-2-carbonsäure-[1,3-di-(5Z,8Z,11Z,14Z-eicosatetraenoyloxy)-2-propyl]ester;
1-[N-(1S-Menthyloxycarbonyl-3-phenylpropyl)-S-alanyl]-(2S,3aR,7aS)-octahydro[1H]indol-2-carbonsäure-[1,3-di(hexadecanoyloxy)-2-propyl]ester;
1-[N-(1S-[1,3-Di(tetradecanoyloxy)-2-propyloxycarbonyl]-3-phenylpropyl)-S-alanyl]-(2S,3aR,7aS)-octahydro-[1H]indol-2-carbonsäure-[1,3-di(tetradecanoyloxy)-2-propyl]ester;
1-[N-(1S-Carboxy-3-phenylpropyl)-S-alanyl]-(2S,3aS,7aS)-octahydro[1H]indol-2-carbonsäure-[1,3-di-(hexadecanoyloxy)-2-propyl]ester;
1-[N-(1S-Ethoxycarbonyl-3-phenylpropyl)-S-alanyl]-(2S,3aS,7aS)-octahydro[1H]indol-2-carbonsäure-[1,3-di-(9Z,12Z,14Z-octadecatrienoyloxy)-2-propyl]ester;
1-[N-[1S-[1,3-Di(octadecanoyloxy)-2-propyloxy-carbonyl]-3-phenylpropyl]-S-alanyl]-(2S,3aS,7aS)-octahydro-[1H]indol-2-carbonsäure-[1,3-di(hexadecanoyloxy)-2-propyl]ester;
1-[N-[1S-[1,3-Di(octadecanoyloxy)-2-propyloxy-carbonyl]-3-phenylpropyl]-S-alanyl-(2S,3aS,7aS)-octahydro-[1H]indol-2-carbonsäure;
1-[N-[1S-[1,3-Di(9Z-octadecenoyloxy)-2-propyloxycarbonyl]-3-phenylpropyl]-S-alanyl]-(2S,3aS,7aS)-octahydro-[1H]indol-2-carbonsäurebenzyhydrylester;
2-[N-(1S-Carboxy-3-phenylpropyl)-S-alanyl]-(1S,3S,5S)-2-azabicyclo[3.3.0]octan-3-carbonsäure-[1,3-di-(5Z,8Z,11Z,14Z-eicosatetraenoyloxy)-2-propyl]ester;
2-[N-[1S-[1,3-Di(hexadecanoyloxy)-2-propyloxycarbonyl]-3-phenylpropyl]-S-alanyl]-(1S,3S,5S)-2-azabicyclo-[3.3.0]-octan-3-carbonsäureoctylester;
2-[N-[1S-[1,3,Di(octadecanoyloxy)-2-propyloxycarbonyl]-3-phenylpropyl]-S-alanyl]-(1S,3S,5S)-2-azabicyclo-[3.3.0]-octan-3-carbonsäure;
2-[N-[1S-[1,3-Di(9Z,12Z-octadecadienoyloxy-2-propyloxycarbonyl]-3-phenylpropyl]-S-alanyl]-(1S,3S,5S)-2-azabicyclo[3.3.0]-octan-3-carbonsäuredecylester;
1-[N-(1S-Ethoxycarbonyl-3-phenylpropyl]-S-alanyl]-3'S-spiro[bicyclo[2.2.2]octan)-2,3'-pyrrolidin]-5'S-yl-carbonsäure[1,3-dihexadecanoyloxy-2-propyl]ester;
1-[N-(1S-Octyloxycarbonyl-3-phenylpropyl]-S-alanyl]-3'S- spiro[(bicyclo[2.2.2]octan)-2,3'-pyrrolidin]-5'S-yl-carbonsäure-(1-octadecanoyloxy-3-octadecanyloxy-2-propyl)-ester;
1-[N-(1S-Carboxy-3-phenylpropyl]-S-alanyl]-3'S-spiro-[2.2.2]octan)-2,3'-pyrrolidin]-5'S-yl-carbonsäure-[1,3-di(9Z,12Z,15Z-octadecatrienoyloxy)-2-propyl]ester;
1-[N-[1S-[1,3-Di(tetradecanoyloxy)-2-propyloxycarbonyl]-3-phenylpropyl]-S-alanyl]-3'S-spiro[(bicyclo[2.2.2]-octan)-2,3'pyrrolidin]-5'S-yl-carbonsäure-[1,3-di(tetradecanoyloxy)-2-propyl]ester;
1-[N-[1S-[1,3-Di(octadecanoyloxy)-2-propyloxycarbonyl]-3-phenylpropyl]-S-alanyl]-3'S-spiro[(bicyclo[2.2.2]-octan)-2,3'pyrrolidin]-5'S-yl-carbonsäuredecylester;
2-[N-(1S-Ethoxycarbonyl-3-phenylpropyl]-S-alanyl]-(1S,3S,5S)-2-azabicyclo[3.3.0]-7-octen-3-carbonsäure-[1,3-di(hexadecanoyloxy)-2-propyl]ester;
2-[N-(1S-Carboxy-3-phenylpropyl]-S-alanyl]-(1S,3S,5S)-2-azabicyclo[3.3.0]-7-octen-3-carbonsäure-(1-hexadecanoyloxy)-3-tetradecanoyloxy-2-propyl)ester;
2-[N-(1S-Nonyloxycarbonyl-3-phenylpropyl]-S-alanyl]-(1S,3S,5S)-2-azabicyclo[3.3.0]-7-octen-3-carbonsäure-(1,3-di(9Z-octadecenoyloxy)-2-propyl)ester;
2-[N-(1S-[1,3-Di(hexadecanoyloxy]-2-propyloxycarbonyl]-3-phenylpropyl]-S-alanyl]-(1S,3S,5S)-2-azabicyclo-[3.3.0]-7-octen-3-carbonsäure-[1,3-di-(hexadecanoyloxy)-2-propyl]-ester;
2-[N-[1S-[1,3-Di(octadecanoyloxy)-2-propyloxycarbonyl]-3-phenylpropyl]-S-alanyl]-(1S,3S,5S)-2-azabicyclo-[3.3.0]-7-octen-3-carbonsäurebenzyhydrylester;
1-[N-(1S-Ethoxycarbonyl-3-phenylpropyl)-S-alanyl]-pyrrolidin-2S-carbonsäure-[1,3-di(hexadecanoyloxy)-2-propyl]ester;
1-[N-(1S-Carboxy-3-phenylpropyl-S-alanyl]-pyrrolidin-2S-carbonsäure-[1,3-di(5Z,8Z,11Z,14Z-eicosatetraenoyloxy)-2-propyl]ester;
1-[N-[1S-[1,3-Di-(eicosanoyloxy)-2-propyloxycarbonyl]-3-phenylpropyl-S-alanyl]-pyrrolidin-2S-carbonsäure-[1,3-di-(eicosanoyloxy)-2-propyl]ester;
1-[N-[1S-[1,3-di(tetradecanoyloxy)-2-propyloxycarbonyl]-3-phenylpropyl-S-lysyl]-pyrrolidin-2S-carbonsäure-(5-nonyl)-ester;
1-[N-[1S-[1,3-Di(hexadecanoyloxy-2-propyloxycarbonyl]-3-phenylpropyl-S-lysyl]-pyrrolidin-2S-carbonsäureundecylester.

## Ansprüche

1. Verbindung der Formel I

$$R^3OOC-\overset{*}{\underset{R^4}{CH}}-\underset{R^5}{N}-\underset{O}{\overset{\parallel}{C}}-\overset{*}{\underset{R^1}{CH}}-NH-\overset{*}{\underset{COOR_2}{CH}}-(CH_2)_n-R \qquad (I)$$

in welcher

n = 1 oder 2 ist,
R = Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten alicyclischaliphatischen Rest mit 7 - 14 C-Atomen,
einen gegebenenfalls substituierten heteroaromatischen oder heteroaromatisch-($C_1$-$C_8$)-aliphatischen Rest mit 5 - 12 Ringatomen,
oder
einen Rest $OR^a$ oder $SR^a$ bedeutet, worin
$R^a$ für einen gegebenenfalls substituierten aliphatischen Rest mit 1 - 4 C-Atomen, für einen gegebenenfalls substituierten aromatischen Rest mit 6 - 12 C-Atomen oder einen gegebenenfalls substituierten heteroaromatischen Rest mit 5 - 12 Ringatomen steht,
$R^1$ Wasserstoff,
einen gegebenenfalls substituierten aliphatischen Rest mit 1 - 21 C-Atomen,
einen gegebenenfalls substituierten alicyclischen Rest mit 3- 20 C-Atomen,
einen gegebenenfalls substituierten alicyclischaliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten heteroaromatischen oder heteroaromatisch-($C_1$-$C_8$)-aliphatischen Rest mit 5 - 12 Ringatomen oder, falls nicht von vorstehenden Definitionen schon umfaßt, die erforderlichenfalls geschützte Seitenkette einer natürlich vorkommenden α-Aminosäure bedeutet,
$R^2$ und $R^3$ gleich oder verschieden sind und Wasserstoff, einen gegebenenfalls substituierten aliphatischen Rest mit 1 - 21 C-Atomen, einen gegebenenfalls substituierten alicyclischen Rest mit 3- 20 C-Atomen, einen gegebenenfalls substituierten aromatischen Rest mit 6 - 12 C-Atomen,
einen gegebenenfalls substituierten araliphatischen Rest mit 7 - 32 C-Atomen,
einen Rest der Formel

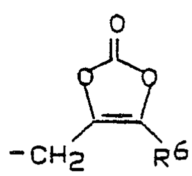

worin $R^6$ Wasserstoff, ein aliphatischer Rest mit 1 - 6 C-Atomen oder ein gegebenenfalls substituierter aromatischer Rest mit 6 -12 C-Atomen ist,
einen Rest der Formel

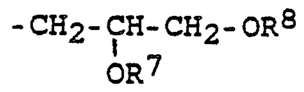 oder 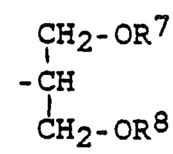

worin $R^7$ und $R^8$ gleich oder verschieden unabhängig voneinander Wasserstoff, einen gegebenenfalls substituierten unverzweigten oder verzweigten, gesättigten oder ungesättigten Alkylrest mit 1 - 23 C-Atomen oder einen gegebenenfalls substituierten, unverzweigten oder verzweigten, gesättigten oder ungesättigten Acylrest mit 1 - 23 C-Atomen bedeuten,
bedeuten,
wobei mindestens einer der Reste $R^2$ oder $R^3$ einen Rest der Formel

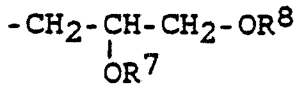 oder 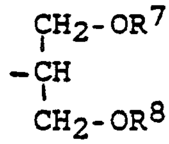

bedeutet, und

$R^4$ und $R^5$ zusammen mit den sie tragenden Atomen ein mono-, bi- oder tricyclisches heterocyclisches Ringsystem mit 3 bis 15 Ring-C-Atomen bilden vorzugsweise aus der Gruppe Tetrahydroisochinolin; Decahydroisochinolin; Octahydroindol; Octahydrocyclopenta-[b]pyrrol; 2-Azaspiro[4.5] decan; 2-Azaspiro-[4.4]-nonan; Spiro [(bicyclo[2.2.1]heptan)-2,3′-pyrrolidin]; Spiro[(bicyclo[2.2.2]octan)-2,3′-pyrrolidin]; 2-Azatricyclo[4.3.0.1$^{6,9}$]decan; Decahydrocyclohepta[b]pyrrol; Hexahydrocyclopropa[b]pyrrol; Octahydroisoindol; Octahydrocyclopenta-[c]pyrrol; 2,3,3a,4, 5,7a-Hexahydroindol; 1,2,3,3a,4,6a-Hexahydrocyclopenta[b]-pyrrol; Pyrrolidin; Indolin; Thiazolidin; die alle gegebenenfalls substituiert sein können,

oder deren physiologisch verträgliche Salze mit Säuren und Basen.

2. Verbindung der Formel I gemäß Anspruch 1, in welcher

a) n = 1 oder 2 ist;

b) R 1. Wasserstoff bedeutet;

2. Alkyl mit 1 - 18 C-Atomen bedeutet;

3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;

4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen;

5. Aryl mit 6 - 12 C-Atomen bedeutet, das durch ($C_1$-$C_8$)-Alkyl, ($C_1$-$C_4$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Alkanoylamino, Methylendioxy, Carboxy, Cyano und/oder Sulfamoyl, mono-, di- oder trisubstituiert sein kann;

6. falls n = 2 ist, ($C_6$-$C_{12}$)-Aryl-($C_1$-$C_8$)-alkyl oder Di-($C_6$-$C_{12}$)-aryl-($C_1$-$C_8$)-alkyl bedeutet, die im Arylteil jeweils wie unter b) 5. beschrieben substituiert sein können; oder

7. Alkoxy mit 1 - 4 C-Atomen;

8. Aryloxy mit 6 - 12 C-Atomen,

das wie unter b) 5. beschrieben substituiert sein kann;

9. mono-bzw. bicyclisches Heteroaryloxy oder Heteroaryl-($C_1$-$C_8$)-alkyl mit 5 - 7 bzw. 8 - 10 Ringatomen, wovon bis zu 9 Ringatome Kohlenstoff und 1 bis 2 Ringatome Schwefel oder Sauerstoff und/oder 1 bis 4 Ringatome Stickstoff darstellen, die im Heteroaryl wie unter b) 5. beschrieben substituiert sein können;

10. Amino-($C_1$-$C_8$)-alkyl;

11. ($C_1$-$C_4$)-alkanoylamino-($C_1$-$C_8$)-alkyl;

12. ($C_7$-$C_{13}$)-Aroylamino-($C_1$-$C_8$)-alkyl;

13. ($C_1$-$C_4$)-Alkoxy-carbonylamino-($C_1$-$C_8$)-alkyl;

14. ($C_6$-$C_{12}$)-Aryl-($C_1$-$C_4$)-alkoxycarbonylamino-($C_1$-$C_8$)-alkyl;

15. ($C_6$-$C_{12}$)-Aryl-($C_1$-$C_4$)-alkylamino-($C_1$-$C_8$)-alkyl;

16. ($C_1$-$C_4$)-Alkylamino-($C_1$-$C_8$)-alkyl;

17. Di-($C_1$-$C_4$)-alkylamino-($C_1$-$C_8$)-alkyl;

18. Guanidino-($C_1$-$C_8$)-alkyl;

19. Imidazolyl;

20. Indolyl;

21. ($C_1$-$C_4$)-Alkylthio;

22. falls n = 2 ist, ($C_1$-$C_4$)-Alkylthio-($C_1$-$C_8$)-alkyl;

23. ($C_6$-$C_{12}$)-Arylthio-($C_1$-$C_8$)-alkyl,

das im Arylteil wie unter b) 5. beschrieben, substituiert sein kann;

24. ($C_6$-$C_{12}$)-Aryl-($C_1$-$C_8$)-alkylthio,

das im Arylteil wie unter b) 5. beschrieben substituiert sein kann;

25. falls n = 2 ist, Carboxy-($C_1$-$C_8$)-alkyl;

26. Carboxy;

27. Carbamoyl;

28. falls n = 2 ist, Carbamoyl-($C_1$-$C_8$)-alkyl;

29. $(C_1-C_4)$-Alkoxy-carbonyl-$(C_1-C_8)$-alkyl;

30. falls n = 2 ist, $(C_6-C_{12})$-Aryloxy-$(C_1-C_8)$-alkyl, das im Arylteil wie unter b) 5. beschrieben substituiert sein kann; oder

31. $(C_6-C_{12})$-Aryl-$(C_1-C_8)$-alkoxy,

das im Arylteil wie unter b) 5. beschrieben substituiert sein kann, bedeutet;

c) $R^1$ 1. Wasserstoff bedeutet;

2. Alkyl mit 1 - 18 C-Atomen bedeutet;

3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;

4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen; oder

5. Aryl mit 6 - 12 C-Atomen,

das wie unter b) 5. beschrieben substituiert sein kann;

6. $(C_6-C_{12})$-Aryl-$(C_1-C_8)$-alkyl oder $(C_7-C_{13})$-Aroyl-$(C_1-C_8)$-alkyl,

die beide im Arylteil wie unter b) 5. beschrieben substituiert sein können;

7. mono- bzw. bicyclisches, gegebenenfalls teilhydriertes Heteroaryl oder Heteroaryl-$(C_1-C_8)$-alkyl mit 5 - 7 bzw. 8 - 10 Ringatomen, wovon bis zu Ringatome Kohlenstoff und 1 bis 2 Ringatome Schwefel-oder Sauerstoffatome und/oder 1 bis 4 Ringatome Stickstoffatome darstellen, die im Heteroaryl wie bei Aryl unter b) 5. beschrieben substituiert sein können; oder

8. falls von c) 1. - 7. noch nicht umfaßt, die gegebenenfalls geschützte Seitenkette einer natürlich vorkommenden α-Aminosäure der Formel $R^1$-CH($NH_2$)-COOH bedeutet;

d) $R^2$ und $R^3$ gleich oder verschieden sind und

1. Wasserstoff bedeuten;

2. Alkyl mit 1 - 18 C-Atomen bedeuten;

3. einen aliphatischen acyclischen Rest der Formel $C_aH_{(2a-b+1)}$ bedeuten, worin Doppelbindungen, falls ihre Zahl 1 übersteigt, nicht kumuliert sind, a für eine ganze Zahl 2 bis 18 und b für eine gerade Zahl 2 bis a stehen;

4. einen mono-, di-, tri-, tetra- oder pentacyclischen, nicht aromatischen Kohlenwasserstoffrest der Formerl $C_cH_{(2c-d-1)}$ bedeuten, der gegebenenfalls verzweigt ist, worin c für eine ganze Zahl 3 bis 20 und d für eine gerade Zahl 0 bis (c-2) stehen; oder

5. Di-$(C_1-C_4)$-alkylamino-$(C_1-C_8)$-alkyl;

6. $(C_1-C_5)$-Alkanoyloxy-$(C_1-C_8)$-alkyl;

7. $(C_1-C_6)$-Alkoxy-carbonyloxy-$(C_1-C_8)$-alkyl;

8. $(C_7-C_{13})$-Aroyloxy-$(C_1-C_8)$-alkyl;

9. $(C_6-C_{12})$-Aryloxycarbonyloxy$(C_1-C_8)$-alkyl;

10. Aryl mit 6 - 12 C-Atomen;

11. $(C_7-C_{20})$-Aralkyl;

12. Phthalidyl;

13. einen Rest der Formel

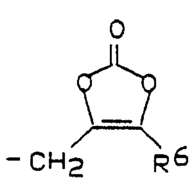

worin $R^6$ Wasserstoff, $(C_1-C_6)$-Alkyl oder Aryl mit 6 - 12-Atomen ist,

14. einen Rest der Formel

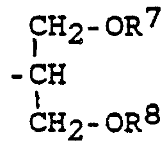

bedeuten, worin $R^7$ und $R^8$ gleich oder verschieden unabhängig voneinander Wasserstoff, einen gegebenenfalls substituierten, unverzweigten oder verzweigten, gesättigten oder ungesättigten Alkylrest mit 1- 23 C-Atomen oder einen gegebenenfalls substituierten, unverzweigten oder verzweigten, gesättigten oder ungesättigten Acylrest mit 1 - 23 C-Atomen bedeuten,
wobei die unter d) 8., 9., 10., 11. und 12. genannten Reste wie unter b) 5. beschrieben im Arylteil substituiert sein können; und wobei mindestens einer der Reste $R^2$ oder $R^3$ einen Rest der Formel

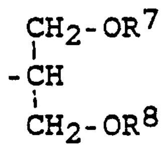

bedeutet, und
e) $R^4$ und $R^5$ zusammen mit den sie tragenden Atomen ein mono-, bi- oder tricyclisches heterocyclisches Ringsystem mit 3 bis 15 Ring-C-Atomen bilden;
oder deren physiologisch verträgliche Salze mit Säuren und Basen.

3. Verbindung der Formerl I gemäß Anspruch 1 oder 2, in welcher
n = 1 oder 2 ist
R = Wasserstoff,
Alkyl mit 1 - 8 C-Atomen,
Alkenyl mit 2 - 6 C-Atomen,
Cycloalkyl mit 3 - 9 C-Atomen,
Aryl mit 6 - 12 C- Atomen,
das durch $(C_1-C_4)$-Alkyl, $(C_1-C_4)$-Alkoxy, Hydroxy, Halogen, Nitro, Amino, Aminomethyl, $(C_1-C_4)$-Alkylamino, Di-$(C_1-C_4)$-alkylamino, $(C_1-C_4)$-Alkanoylamino, Methylendioxy, Carboxy, Cyano und/oder Sulfamoyl, mono-, di- oder trisubstituiert sein kann,
Alkoxy mit 1 - 4 C-Atomen,
Aryloxy mit 6 - 12 C-Atomen,
das wie oben bei Aryl beschrieben substituiert sein kann,
mono- bzw. bicyclisches Heteroaryloxy mit 5 - 7 bzw. 8 - 10 Ringatomen, wovon 1 bis 2 Ringatome Schwefel-oder Sauerstoffatome und/oder 1 bis 4 Ringatome Stickstoff darstellen,
das wie oben bei ARyl beschrieben substituiert sein kann,
Amino-$(C_1-C_4)$-alkyl,
$(C_1-C_4)$-Alkanoylamino-$(C_1-C_4)$alkyl,
$(C_7-C_{13})$-Aroylamino-$(C_1-C_4)$-alkyl,
$(C_1-C_4)$-Alkoxy-carbonylamino-$(C_1-C_4)$-alkyl,
$(C_6-C_{12})$-Aryl-$(C_1-C_4)$-alkoxycarbonylamino-$(C_1-C_4)$-alkyl,
$(C_6-C_{12})$-Aryl-$(C_1-C_4)$-alkylamino-$(C_1-C_4)$-alkyl,
$(C_1-C_4)$-Alkylamino-$(C_1-C_4)$-alkyl,
Di-$(C_1-C_4)$-alkylamino-$(C_1-C_4)$-alkyl,
Guanidino-$(C_1-C_4)$-alkyl,
Imidazolyl, Indolyl,
$(C_1-C_4)$-Alkylthio,
$(C_1-C_4)$-Alkylthio-$(C_1-C_4)$-alkyl,
$(C_6-C_{12})$-Arylthio-$(C_1-C_4)$-alkyl,
das im Arylteil wie oben bei Aryl beschrieben, substituiert sein kann,
$(C_6-C_{12})$-Aryl-$(C_1-C_4)$-alkylthio,
das im Arylteil wie oben bei Aryl beschrieben substituiert sein kann,
Carboxy-$(C_1-C_4)$-alkyl,

Carboxy, Carbamoyl,
Carbamoyl-($C_1$-$C_4$)-alkyl,
($C_1$-$C_4$)-Alkoxy-carbonyl-($C_1$-$C_4$)-alkyl,
($C_6$-$C_{12}$)-Aryloxy-($C_1$-$C_4$)-alkyl,
das im Arylteil wie oben bei Aryl beschrieben substituiert sein kann oder
($C_6$-$C_{12}$)-Aryl-($C_1$-$C_4$)-alkoxy,
das im Arylteil wie oben bei Aryl beschrieben substituiert sein kann,
$R^1$ Wasserstoff,
Alkyl mit 1 - 6 C-Atomen,
Alkenyl mit 2 - 6 C-Atomen,
Alkinyl mit 2 - 6 C-Atomen,
Cycloalkyl mit 3 - 9 C-Atomen,
Cycloalkenyl mit 5 - 9 C-Atomen,
($C_3$-$C_9$)-Cycloalkyl-($C_1$-$C_4$)-alkyl,
($C_5$-$C_9$)-Cycloalkenyl-($C_1$-$C_4$)-alkyl,
gegebenenfalls teilhydriertes Aryl mit 6 - 12 C-Atomen, das wie oben bei R beschrieben substituiert sein kann, ($C_6$-$C_{12}$)-Aryl-($C_1$-$C_4$)-alkyl oder ($C_7$-$C_{13}$)-Aroyl-($C_1$ oder $C_2$)-alkyl
die beide wie das vorstehende Aryl substituiert sein können
mono- bzw. bicyclisches, gegebenenfalls teilhydriertes Heteroaryl mit 5 - 7 bzw. 8 - 10 Ringatomen, wovon 1 bis 2 Ringatome Schwefel- oder Sauerstoffatome und/oder
1 bis 4 Ringatome Stickstoffatome darstellen, das wie das vorstehende Aryl substituiert sein kann oder
die gegebenenfalls geschützte Seitenkette einer natürlichen vorkommenden α-Aminosäure $R^1$-CH($NH_2$)-COOH bedeutet,
$R^2$ und $R^3$ gleich oder verschieden sind und
Wasserstoff,
Alkyl mit 1 - 6 C-Atomen,
Alkenyl mit 2 - 6 C-Atomen,
Di-($C_1$-$C_4$)-alkylamino-($C_1$-$C_4$)-alkyl,
($C_1$-$C_5$)-Alkanoyloxy-($C_1$-$C_4$)-alkyl,
($C_1$-$C_6$)-Alkoxy-carbonyloxy-($C_1$-$C_4$)-alkyl,
($C_7$-$C_{13}$)-Aroyloxy-($C_1$-$C_4$)-alkyl,
($C_6$-$C_{12}$)-Aryloxycarbonyloxy($C_1$-$C_4$)-alkyl,
Aryl mit 6 - 12 C-Atomen,
($C_6$-$C_{12}$)-Aryl-($C_1$-$C_4$)-alkyl,
($C_3$-$C_9$)-Cycloalkyl,
($C_3$-$C_9$)-Cycloalkyl-($C_1$-$C_4$)-alkyl,
Phthalidyl,
einen Rest der Formel

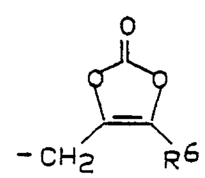

worin $R^5$ Wasserstoff, ($C_1$-$C_6$)-Alkyl oder Aryl mit 6 - 12 C-Atomen ist,
einen Rest der Formel

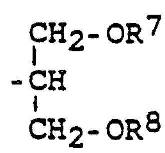

bedeuten, worin $R^7$ und $R^8$ gleich oder verschieden unabhängig voneinander Wasserstoff oder einen gegebenenfalls substituierten, unverzweigten oder verzweigten, gesättigten oder ungesättigten Acylrest mit

1 - 23 C-Atomen bedeuten,
und wobei mindestens einer der Reste $R^2$ oder $R^3$ einen Rest der Formel

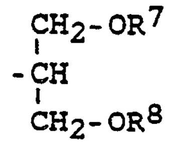

bedeutet, und
$R^4$ und $R^5$ die oben angegebene Bedeutung haben oder der physiologisch verträgliche Salze mit Säuren und Basen.

4. Verbindung der Formel I gemäß einem oder mehrerer der Ansprüche 1 bis 3, in welcher
n = 1 oder 2 ist,
R $(C_1-C_6)$-Alkyl, $(C_2-C_6)$-Alkenyl, $(C_3-C_9)$-Cycloalkyl, Amino-$(C_1-C_4)$-alkyl, $(C_2-C_5)$-Acylamino-$(C_1-C_4)$-alkyl, $(C_7-C_{13})$-Aroylamino-$(C_1-C_4)$-alkyl, $(C_1-C_4)$-Alkoxy-carbonylamino-$(C_1-C_4)$-alkyl, $(C_6-C_{12})$-Aryl-$(C_1-C_4)$-alkoxycarbonylamino-$(C_1-C_4)$-alkyl, $(C_6-C_{12})$-Aryl, das durch $(C_1-C_4)$-Alkyl, $(C_1-C_4)$-Alkoxy, Hydroxy, Halogen, Nitro, Amino, $(C_1-C_4)$-Alkylamino, Di-$(C_1-C_4)$-alkylamino und/oder Methylendioxy mono-, di- oder trisubstituiert sein kann, oder 3-Indolyl, insbesondere Methyl, Ethyl, Cyclohexyl, tert. Butoxycarbonylamino-$(C_1-C_4)$-alkyl, Benzoyloxycarbonylamino-$(C_1-C_4)$-alkyl oder Phenyl, das durch Phenyl, $(C_1-C_2)$-Alkyl, ($C_1$ oder $C_2$)-Alkoxy, Hydroxy, Fluor, Chlor, Brom, Amino, $(C_1-C_4)$-Alkylamino, Di-$(C_1-C_4)$alkylamino, Nitro und/oder Methylendioxy mono- oder disubstituiert oder im Falle von Methoxy, trisubstituiert sein kann, bedeutet,
$R^1$ Wasserstoff oder $(C_1-C_6)$-Alkyl, das gegebenenfalls durch Amino, $(C_1-C_6)$-Acylamino oder Benzoylamino substituiert sein kann, $(C_2-C_6)$-Alkenyl, $(C_3-C_9)$-Cycloalkyl, $(C_5-C_9)$-Cycloakenyl, $(C_3-C_7)$-Cycloalkyl-$(C_1-C_4)$-alkyl, $(C_6-C_{12})$-Aryl oder teilhydriertes Aryl, das jeweils durch $(C_1-C_4)$-Alkyl, ($C_1$ oder $C_2$)-Alkoxy oder Halogen substituiert sein kann, $(C_6-C_{12})$-aryl-($C_1$ bis $C_4$)-alkyl oder $(C_7-C_{13})$-Aroyl-$(C_1-C_2)$-alkyl, die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure, insbesondere aber Wasserstoff, $(C_1-C_3)$-Alkyl, ($C_2$ oder $C_3$)-Alkenyl, die gegebenenfalls geschützte Seitenkette von Lysin, Phenylalanin oder Tyrosin, Benzyl, 4-Methoxybenzyl, 4-Ethoxybenzyl, Phenethyl, 4-Amino-butyl oder Benzoylmethyl bedeutet,
$R^2$ und $R^3$ gleiche oder verschiedene Reste Wasserstoff, $(C_1-C_6)$-Alkyl, $(C_2-C_6)$-Alkenyl, $(C_6-C_{12})$-Aryl-$(C_1-C_4)$-alkyl, $(C_1-C_5)$-Alkanoyloxy-$(C_1-C_4)$-alkyl, $(C_1-C_6)$-Alkoxycarbonyloxy-$(C_1-C_4)$-alkyl, $(C_7-C_{13})$-Aroyloxy-$(C_1-C_4)$-alkyl, $(C_6-C_{12})$-Aryloxycarbonyloxy-$(C_1-C_4)$alkyl, Phthalidyl, einen Rest der Formel

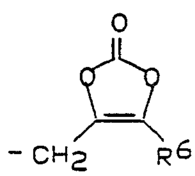

worin $R^6$ Wasserstoff, $(C_1-C_6)$-Alkyl oder Aryl mit 6 - 12 C-Atomen ist, oder einen Rest der Formel

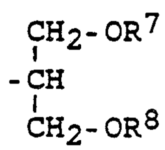

worin $R^7$ und $R^8$ Wasserstoff oder einen gesättigten oder ungesättigten Acylrest mit 8 - 23 C-Atomen bedeuten,
und wobei mindestens einer der Reste $R^2$ oder $R^3$ einen Rest der Formel

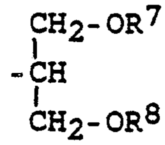

bedeutet und

$R^4$ und $R^5$ die oben angegebene Bedeutung haben oder deren physiologisch verträgliche Salze mit Säuren und Basen.

5. Verfahren zur Herstellung einer Verbindung der Formel I gemäß einem der Ansprüche 1 bis 4, dadurch gekennzeichnet, daß man

a) eine Verbindung der Formel II

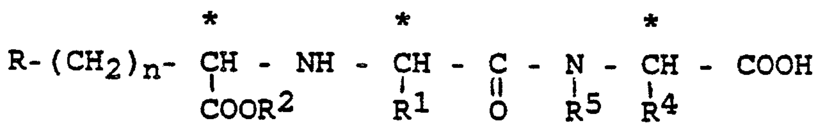 (II)

in der R, $R^1$, $R^2$, $R^4$, $R^5$ und n die gleiche Bedeutung wie in Formel I haben, mit einer Verbindung der Formel IIIa/b

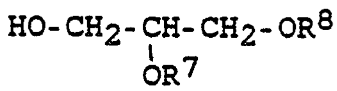

(IIIa)

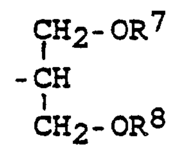

(IIIb)

in der $R^7$ und $R^8$ die gleiche Bedeutung wie in Formel I besitzen, verestert,

oder daß man

b) eine Verbindung der Formel II, in der R, $R^1$, $R^2$, $R^4$, $R^5$ und n die gleiche Bedeutung wie in Formel I haben, mit einer Verbindung der Formel IVa/b

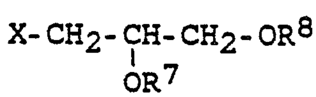

(IVa)

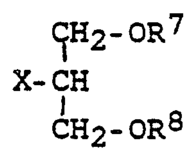

(IVb)

in der $R^7$ und $R^8$ die gleiche bedeutung wie in Formel I haben, und in der X eine Abgangsgruppe, die nucleophil verdrängt werden kann, insbesondere ein Cl, Br, J-Atom oder einen Sulfonsäurerest, bedeutet, unter Bedingungen einer nucleophilen Substitution umsetzt,

oder daß man

c) eine Verbindung der Formel V

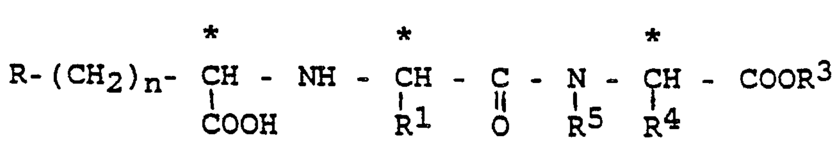 (V)

in der R, $R^1$, $R^3$, $R^4$, $R^5$ und n die gleiche Bedeutung wie in Formel I haben, mit einer Verbindung der Formel IIIa/b umsetzt,
oder daß man
d) eine Verbindung der Formel V, in der R, $R^1$, $R^3$, $R^4$, $R^5$ und n die gleiche Bedeutung wie in Formel I haben, mit einer Verbindung der Formel IVa/b, umsetzt,
oder daß man
e) eine Verbindung der Formel VI

$$R-(CH_2)_n-\underset{\underset{COOR^2}{|}}{\overset{*}{CH}}-OSO_2CF_3 \qquad (VI)$$

worin R, $R^2$ und n die gleiche Bedeutung wie in Formel I haben, mit einer Verbindung der Formel VII

$$R^3OOC-\underset{\underset{R^4}{|}}{\overset{*}{CH}}-\underset{\underset{R^5}{|}}{N}-\underset{\underset{O}{\|}}{C}-\underset{\underset{R^1}{|}}{\overset{*}{CH}}-NH_2 \qquad (VII)$$

worin $R^1$, $R^3$, $R^4$ und $R^5$ die gleiche bedeutung wie in Formel I haben, umsetzt,
oder daß man
f) eine Verbindung der Formel VII mit einer Verbindung der Formel VIII
$R^2OOC-CH=CH-CO-R$ (VIII)
in welcher R und $R^2$ die gleiche Bedeutung wie in Formel I haben, umsetzt, und die Carbonylgruppe hydriert,
oder daß man
g) eine Verbindung der Formel VII mit einer Verbindung der Formel IX
$R-(CH_2)_n-\underset{\underset{O}{\|}}{C}-COOR^2$ (IX)
worin R, $R^2$ und n die gleiche Bedeutung wie in Formel I haben, umsetzt und die erhaltenen Schiff-Basen reduziert,
oder daß man
h) eine Verbindung der Formel X

$$R-(CH_2)_n-\underset{\underset{COOR^2}{|}}{\overset{*}{CH}}-NH-\underset{\underset{R^1}{|}}{\overset{*}{CH}}-\underset{\underset{O}{\|}}{C}-\underset{\underset{R^5}{|}}{N}-\underset{\underset{R^4}{|}}{\overset{*}{CH}}-COOR^{10} \qquad (X)$$

in welcher R, $R^1$, $R^2$, $R^4$, $R^5$ und n die gleiche Bedeutung wie in Formel I haben und in welcher $R^{10}$ einer Rest der Formel

$$-CH_2-\underset{\underset{OSG^1}{|}}{CH}-CH_2-SG^2 \quad \text{oder} \quad -\underset{\underset{CH_2-O-SG^2}{|}}{\overset{\overset{CH_2-O-SG^1}{|}}{CH}}$$

bedeutet,
worin $SG^1$ und $SG^2$ gleiche oder verschiedene oder auch eine gemeinsame leicht abspaltbare Schutzgruppe einer Hydroxylfunktion bedeuten, durch Abspaltung einer oder beider Schutzgruppen in eine Verbindung der Formel I, inder $R^7$ und/oder $R^8$ Wasserstoff bedeuten überführt und diese gegebenenfalls durch Acylierung zu den Endprodukten der Formel I umsetzt,
oder daß man

i) eine Verbindung der Formel XI

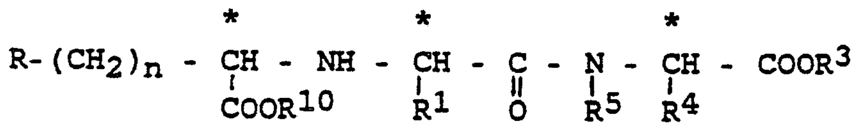

(XI)

in welcher R, $R^1$, $R^2$, $R^4$, $R^5$ und n die gleiche Bedeutung wie in Formel I und $R^{10}$ die gleiche Bedeutung wie in Formel X besitzen, wie unter der Verfahrensvarianten h) beschrieben, zu Endprodukten der Formel I umsetzt,
und die so erhaltenen Verbindungen der Formel I gegebenenfalls in ihre physiologisch verträglichen Salze überführt.

6. Verbindung gemäß einem oder mehrerer der Ansprüche 1 bis 4 zur Anwendung als Heilmittel.

7. Verbindung gemäß einem oder mehrerer der Ansprüche 1 bis 4 zur Anwendung als Heilmittel bei der Behandlung des Bluthochdrucks.

8. Verbindung gemäß einem oder mehrerer der Ansprüche 1 bis 4 zur Anwendung als Heilmittel bei der Behandlung cognitiver Dysfunktionen.

9. Pharmazeutisches Mittel enthaltend eine Verbindung gemäß einem oder mehrerer der Ansprüche 1 bis 4.

10. Verfahren zur Herstellung eines Mittels gemäß Anspruch 9, dadurch gekennzeichnet, daß man eine Verbindung der Formel I oder deren physiologisch verträgliches Salz und einen physiologisch annehmbaren Träger gegebenenfalls zusammen mit weiteren Zusatz- oder Hilfsstoffen in eine geeignete Darreichungsform bringt.

Patentansprüche für folgende Vertragsstaaten : ES, GR

1. Verfahren zur Herstellung einer Verbindung der Formel I

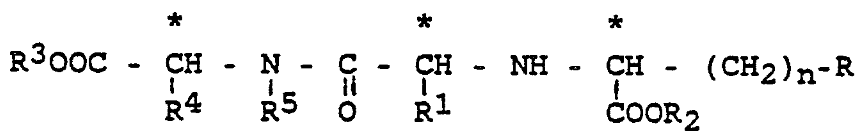

(I)

in welcher
n = 1 oder 2 ist,
R = Wasserstoff,
einen gegebenenfalls substituierten aliphatischen Rest mit 1 - 21 C-Atomen,
einen gegebenenfalls substituierten alicyclischen Rest mit 3 - 20 C-Atomen,
einen gegebenenfalls substituierten aromatischen Rest mit 6 - 12 C-Atomen,
einen gegebenenfalls substituierten araliphatischen Rest mit 7 - 32 C-Atomen,
einen gegebenenfalls substituierten alicyclisch-aliphatischen Rest mit 7 - 14 C-Atomen,
einen gegebenenfalls substituierten heteroaromatischen oder heteroaromatisch-($C_1$-$C_8$)-aliphatischen Rest mit 5 - 12 Ringatomen, oder
einen Rest $OR^a$ oder $SR^a$ bedeutet, worin
$R^a$ für einen gegebenenfalls substituierten aliphatischen Rest mit 1 - 4 C-Atomen, für einen gegebenenfalls substituierten aromatischen Rest mit 6 - 12 C-Atomen oder einen gegebenenfalls substituierten heteroaromatischen Rest mit 5 - 12 Ringatomen steht,
$R^1$ Wasserstoff,
einen gegebenenfals substituierten aliphatischen Rest mit 1 - 21 C-Atomen,
einen gegebenenfalls substituierten alicyclischen Rest mit 3 - 20 C-Atomen,
einen gegebenenfalls substituierten alicyclisch-aliphatischen Rest mit 4 - 20 C-Atomen,
einen gegebenenfalls substituierten aromatischen Rest mit 6 - 12 C-Atomen,
einen gegebenenfalls substituierten araliphatischen Rest mit 7- 32 C-Atomen,
einen gegebenenfalls substituierten heteroaromatischen oder heteroaromatisch-($C_1$-$C_8$)-aliphatischen Rest mit 5 - 12 Ringatomen oder, falls nicht von vorstehenden Definitionen schon umfaßt, die erforderlichenfalls geschützte Seitenkette einer natürlich vorkommenden α-Aminosäure bedeutet,

$R^2$ und $R^3$ gleich oder verschieden sind und Wasserstoff, einen gegebenenfalls substituierten aliphatischen Rest mit 1 - 21 C-Atomen, einen gegebenenfalls substituierten alicyclischen Rest mit 3 - 20 C-Atomen, einen gegebenenfalls substituierten aromatischen Rest mit 6 - 12 C-Atomen,
einen gegebenenfalls substituierten araliphatischen Rest mit 7 - 32 C-Atomen,
einen Rest der Formel

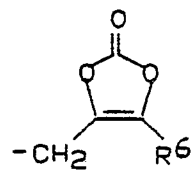

worin $R^6$ Wasserstoff, ein aliphatischer Rest mit 1 - 6 C-Atomen oder ein gegebenenfalls substituierter aromatischer Rest mit 6 -12 C-Atomen ist,
einen Rest der Formel

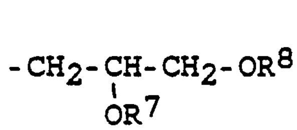 oder 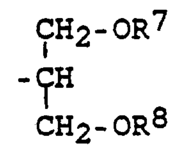

worin $R^7$ und $R^8$ gleich oder verschieden unabhängig voneinander Wasserstoff, einen gegebenenfalls substituierten unverzweigten oder verzweigten, gesättigten oder ungesättigten Alkylrest mit 1 - 23 C-Atomen oder einen gegebenenfalls substituierten, unverzweigten oder verzweigten, gesättigten oder ungesättigten Acylrest mit 1 - 23 C-Atomen bedeuten, bedeuten,
wobei mindestens einer der Reste $R^2$ oder $R^3$ einen Rest der Formel

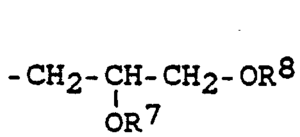 oder 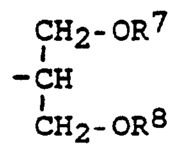

bedeutet, und
$R^4$ und $R^5$ zusammen mit den sie tragenden Atomen ein mono-, bi- oder tricyclisches heterocyclisches Ringsystem mit 3 bis 15 Ring-C-Atomen bilden vorzugsweise aus der Gruppe Tetrahydroisochinolin; Decahydroisochinolin; Octahydroindol; Octahydrocyclopenta-[b]pyrrol; 2-Azaspiro[4.5] decan; 2-Azaspiro-[4.4]-nonan; Spiro [(bicyclo[2.2.1]heptan)-2,3′-pyrrolidin]; Spiro[(bicyclo[2.2.2]octan)-2,3′-pyrrolidin]; 2-Azatricyclo[4.3.0.1$^{6,9}$]decan; Decahydrocyclohepta[b]pyrrol; Hexahydrocyclopropa[b]pyrrol; Octahydroisoindol; Octahydrocyclopenta-[c]pyrrol; 2,3,3a,4, 5,7a-Hexahydroindol; 1,2,3,3a,4,6a-Hexahydrocyclopenta[b]-pyrrol; Pyrrolidin; Indolin; Thiazolidin; die alle gegebenenfalls substituiert sein können,
oder deren physiologisch verträglichem Salz mit Säuren und Basen, dadurch gekennzeichnet, daß man
a) eine Verbindung der Formel II

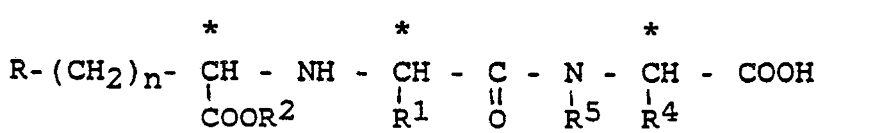 (II)

in der R, $R^1$, $R^2$, $R^4$, $R^5$ und n die gleiche Bedeutung wie in Formel I haben, mit einer Verbindung der Formel IIIa/b

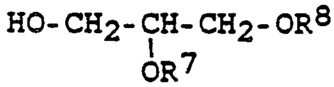

,

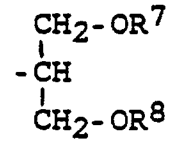

(IIIa) (IIIb)

in der $R^7$ und $R^8$ die gleiche Bedeutung wie in Formel I besitzen, verestert,
oder daß man
b) eine Verbindung der Formel II, in der R, $R^1$, $R^2$, $R^4$, $R^5$ und n die gleiche Bedeutung wie in Formel I haben, mit einer Verbindung der Formel IVa/b

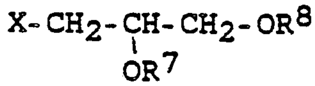

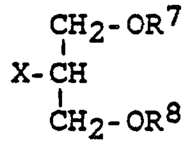

(IVa) (IVb)

in der $R^7$ und $R^8$ die gleiche Bedeutung wie in Formel I haben, und in der X eine Abgangsgruppe, die nucleophil verdrängt werden kann, insbesondere ein Cl, Br, J-Atom oder einen Sulfonsäurerest, bedeutet, unter Bedingungen einer nucleophilen Substitution umsetzt,
oder daß man
c) eine Verbindung der Formel V

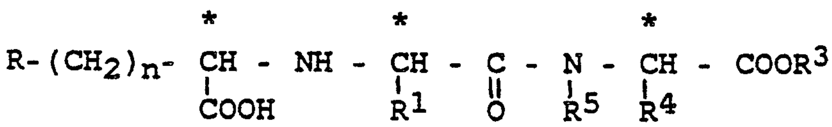

(V)

in der R, $R^1$, $R^3$, $R^4$, $R^5$ und n die gleiche Bedeutung wie in Formel I haben, mit einer Verbindung der Formel IIIa/b umsetzt,
oder daß man
d) eine Verbindung der Formel V, in der R, $R^1$, $R^3$, $R^4$, $R^5$ und n die gleiche Bedeutung wie in Formel I haben, mit einer Verbindung der Formel IVa/b, umsetzt,
oder daß man
e) eine Verbindung der Formel VI

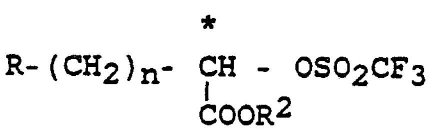

(VI)

worin R, $R^2$ und n die gleiche Bedeutung wie in Formel I haben, mit einer Verbindung der Formel VII

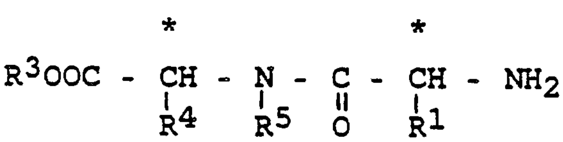

(VII)

worin $R^1$, $R^3$, $R^4$ und $R^5$ die gleiche Bedeutung wie in Formel I haben, umsetzt,
oder daß man

$$R\text{-}(CH_2)_n\text{-}\overset{*}{\underset{\underset{COOR^2}{|}}{CH}}\text{ - }OSO_2CF_3 \qquad (VI)$$

worin R, $R^2$ und n die gleiche Bedeutung wie in Formel I haben, mit einer Verbindung der Formel VII

$$R^3OOC\text{ - }\overset{*}{\underset{\underset{R^4}{|}}{CH}}\text{ - }\underset{\underset{R^5}{|}}{N}\text{ - }\underset{\underset{O}{||}}{C}\text{ - }\overset{*}{\underset{\underset{R^1}{|}}{CH}}\text{ - }NH_2 \qquad (VII)$$

worin $R^1$, $R^3$, $R^4$ und $R^5$ die gleiche Bedeutung wie in Formel I haben, umsetzt,
oder daß man
f) eine Verbindung der Formel VII mit einer Verbindung der Formel VIII
$R^2OOC - CH = CH - CO - R$ (VIII)
in welcher R und $R^2$ die gleiche Bedeutung wie in Formel I haben, umsetzt, und die Carbonylgruppe hydriert,
oder daß man
g) eine Verbindung der Formel VII mit einer Verbindung der Formel IX
$R\text{-}(CH_2)_n\text{ - }\underset{\underset{O}{||}}{C}\text{ - }COOR^2$ (IX)
worin R, $R^2$ und n die gleiche Bedeutung wie in Formel I haben, umsetzt und die erhaltenen Schiff-Basen reduziert,
oder daß man
h) eine Verbindung der Formel X

$$R\text{-}(CH_2)_n\text{ - }\overset{*}{\underset{\underset{COOR^2}{|}}{CH}}\text{ - }NH\text{ - }\overset{*}{\underset{\underset{R^1}{|}}{CH}}\text{ - }\underset{\underset{O}{||}}{C}\text{ - }\underset{\underset{R^5}{|}}{N}\text{ - }\overset{*}{\underset{\underset{R^4}{|}}{CH}}\text{ - }COOR^{10} \qquad (X)$$

in welcher R, $R^1$, $R^2$, $R^4$, $R^5$ und n die gleiche Bedeutung wie in Formel I haben und in welcher $R^{10}$ einen Rest der Formel

$$-CH_2\text{-}\underset{\underset{OSG^1}{|}}{CH}\text{-}CH_2\text{-}SG^2 \quad - \qquad \text{oder} \qquad -\underset{\underset{CH_2\text{-}O\text{-}SG^2}{|}}{\overset{\overset{CH_2\text{-}O\text{-}SG^1}{|}}{CH}}$$

bedeutet,
worin $SG^1$ und $SG^2$ gleiche oder verschiedene oder auch eine gemeinsame leicht abspaltbare Schutzgruppe einer Hydroxylfunktion bedeuten, durch Abspaltung einer oder beider Schutzgruppen in eine Verbindung der Formel I, inder $R^7$ und/oder $R^8$ Wasserstoff bedeuten überführt und diese gegebenenfalls durch Acylierung zu den Endprodukten der Formel I umsetzt, oder daß man
i) eine Verbindung der Formel XI

$$R\text{-}(CH_2)_n\text{ - }\overset{*}{\underset{\underset{COOR^{10}}{|}}{CH}}\text{ - }NH\text{ - }\overset{*}{\underset{\underset{R^1}{|}}{CH}}\text{ - }\underset{\underset{O}{||}}{C}\text{ - }\underset{\underset{R^5}{|}}{N}\text{ - }\overset{*}{\underset{\underset{R^4}{|}}{CH}}\text{ - }COOR^3 \qquad (XI)$$

in welcher R, $R^1$, $R^2$, $R^4$, $R^5$ und n die gleiche Bedeutung wie in Formel I und $R^{10}$ die gleiche Bedeutung

Amino, Aminomethyl, $(C_1\text{-}C_4)$-Alkylamino, Di- $(C_1\text{-}C_4)$-alkylamino, $(C_1\text{-}C_4)$- Alkanoylamino, Methylendioxy, Carboxy, Cyano und/oder Sulfamoyl, mono-, di- oder trisubstituiert sein kann;
6. falls n = 2 ist, $(C_6\text{-}C_{12})$-Aryl-$(C_1\text{-}C_8)$-alkyl oder Di-$(C_6\text{-}C_{12})$-aryl-$(C_1\text{-}C_8)$-alkyl bedeutet, die im Arylteil jeweils wie unter b) 5. beschrieben substituiert sein können; oder
7. Alkoxy mit 1 - 4 C-Atomen;
8. Aryloxy mit 6 - 12 C-Atomen,
das wie unter b) 5. beschrieben substituiert sein kann;
9. mono- bzw. bicyclisches Heteroaryloxy oder Heteroaryl-$(C_1\text{-}C_8)$-alkyl mit 5 - 7 bzw. 8 - 10 Ringatomen, wovon bis zu 9 Ringatome Kohlenstoff und 1 bis 2 Ringatome Schwefel oder Sauerstoff und/oder 1 bis 4 Ringatome Stickstoff darstellen, die im Heteroaryl wie unter b) 5. beschrieben substituiert sein können;
10. Amino-$(C_1\text{-}C_8)$-alkyl;
11. $(C_1\text{-}C_4)$-Alkanoylamino-$(C_1\text{-}C_8)$-alkyl;
12. $(C_7\text{-}C_{13})$-Aroylamino-$(C_1\text{-}C_8)$-alkyl;
13. $(C_1\text{-}C_4)$-Alkoxy-carbonylamino-$(C_1\text{-}C_8)$-alkyl;
14. $(C_6\text{-}C_{12})$-Aryl-$(C_1\text{-}C_4)$-alkoxycarbonylamino-$(C_1\text{-}C_8)$-alkyl;
15. $(C_6\text{-}C_{12})$-Aryl-$(C_1\text{-}C_4)$-alkylamino$(C_1\text{-}C_8)$-alkyl;
16. $(C_1\text{-}C_4)$-Alkylamino-$(C_1\text{-}C_8)$-alkyl;
17. Di-$(C_1\text{-}C_4)$-alkylamino-$(C_1\text{-}C_8)$-alkyl;
18. Guanidino-$(C_1\text{-}C_8)$-alkyl;
19. Imidazolyl;
20. Indolyl;
21. $(C_1\text{-}C_4)$-Alkylthio;
22. falls n = 2 ist, $(C_1\text{-}C_4)$-Alkylthio-$(C_1\text{-}C_8)$-alkyl;
23. $(C_6\text{-}C_{12})$-arylthio-$(C_1\text{-}C_8)$-alkyl,
das im Arylteil wie unter b) 5. beschrieben, substituiert sein kann;
24. $(C_6\text{-}C_{12})$-Aryl-$(C_1\text{-}C_8)$-alkylthio,
das im Arylteil wie unter b) 5. beschrieben substituiert sein kann;
25. falls n = 2 ist, Carboxy-$(C_1\text{-}C_8)$-alkyl;
26. Carboxy;
27. Carbamoyl;
28. falls n = 2 ist, Carbamoyl-$(C_1\text{-}C_8)$-alkyl;
29. $(C_1\text{-}C_4)$-Alkoxy-carbonyl-$(C_1\text{-}C_8)$-alkyl;
30. falls n = 2 ist, $(C_6\text{-}C_{12})$-Aryloxy-$(C_1\text{-}C_8)$-alkyl, das im Arylteil wie unter b) 5. beschrieben substituiert sein kann; oder
31. $(C_6\text{-}C_{12})$-Aryl-$(C_1\text{-}C_8)$-alkoxy,
das im Arylteil wie unter b) 5. beschrieben substituiert sein kann, bedeutet;
c) $R^1$ 1. Wasserstoff bedeutet;
2. Alkyl mit 1 - 18 C-Atomen bedeutet;
3. einen aliphatischen acyclischen Rest der Formel $C_aH_{(2a-b+1)}$ bedeutet, worin Doppelbindungen, falls ihre Zahl 1 übersteigt, nicht kumuliert sind, a für eine ganze Zahl 2 bis 18 und b für eine gerade Zahl 2 bis a stehen;
4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen; oder
5. Aryl mit 6 - 12 C-Atomen,
das wie unter b) 5. beschrieben substituiert sein kann;
6. $(C_6\text{-}C_{12})$-Aryl-$(C_1\text{-}C_8)$-alkyl oder $(C_7\text{-}C_{13})$-Aroyl-$(C_1\text{-}C_8)$-alkyl,
die beide im Arylteil wie unter b) 5. beschrieben substituiert sein können;
7. mono- bzw. bicyclisches, gegebenenfalls teilhydriertes Heteroaryl oder Heteroaryl-$(C_1\text{-}C_8)$-alkyl mit 5 - 7 bzw. 8 - 10 Ringatomen, wovon bis zu 9 Ringatome Kohlenstoff und 1 bis 2 Ringatome Schwefel-oder Sauerstoffatome und/oder 1 bis 4 Ringatome Stickstoffatome darstellen, die im Heteroaryl wie bei Aryl unter b) 5. beschrieben substituiert sein können; oder
8. falls von c) 1. -7. noch nicht umfaßt, die gegebenenfalls geschützte Seitenkette einer natürlich vorkommenden α-Aminosäure der Formel $R^1$-CH($NH_2$)-COOH bedeutet;
d) $R^2$ und $R^3$ gleich oder verschieden sind und
1. Wasserstoff bedeuten;
2. Alkyl mit 1 - 18 C-Atomen bedeutet;
3. einen aliphatischen acyclischen Rest der Formel $C_aH_{(2a-b+1)}$ bedeutet, worin Doppelbindungen, falls ihre

Zahl 1 übersteigt, nicht kumuliert sind, a für eine ganze Zahl 2 bis 18 und b für eine gerade Zahl 2 bis a stehen;

4. einen mono-, di-, tri-, tetra- oder pentacyclischen, nicht aromatischen Kohlenwasserstoffrest der Formel $C_cH_{(2c-d-1)}$ bedeutet, der gegebenenfalls verzweigt ist, worin c für eine ganze Zahl 3 bis 20 und d für eine gerade Zahl 0 bis (c-2) stehen; oder

5. Di-($C_1$-$C_4$)-alkylamino-($C_1$-$C_8$)-alkyl;

6. ($C_1$-$C_5$)-alkanoyloxy-($C_1$-$C_8$)-alkyl;

7. ($C_1$-$C_6$)-Alkoxy-carbonyloxy-($C_1$-$C_8$)-alkyl;

8. ($C_7$-$C_{13}$)-Aroyloxy-($C_1$-$C_8$)-alkyl;

9. ($C_6$-$C_{12}$)-Aryloxycarbonyloxy($C_1$-$C_8$)-alkyl;

10. Aryl mit 6 - 12 C-Atomen;

11. ($C_7$-$C_{20}$)-Aralkyl;

12. Phthalidyl;

13. einen Rest der Formel

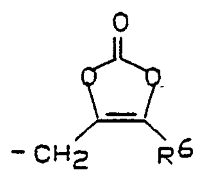

worin $R^6$ Wasserstoff, ($C_1$-$C_6$)-Alkyl oder Aryl mit 6 - 12 C-Atomen ist,

14. einen Rest der Formel

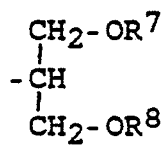

bedeuten, worin $R^7$ und $R^8$ gleich oder verschieden unabhängig voneinander Wasserstoff, einen gegebenenfalls substituierten, unverzweigten oder verzweigten, gesättigten oder ungesättigten Alkylrest mit 1 - 23 C-Atomen oder einen gegebenenfalls substituierten, unverzweigten oder verzweigten, gesättigten oder ungesättigten Acylrest mit 1 - 23 C-Atomen bedeuten,

wobei die unter d) 8., 9., 10., 11. und 12. genannten Reste wie unter b) 5. beschrieben im Arylteil substituiert sein können; und

wobei mindestens einer der Reste $R^2$ oder $R^3$ einen Rest der Formel

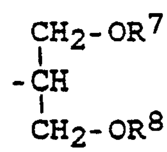

bedeutet, und

e) $R^4$ und $R^5$ zusammen mit den sie tragenden Atomen ein mono-, bi oder tricyclisches heterocyclisches Ringsystem mit 3 bis 15 ring-C-Atomen bilden;

oder deren physiologisch verträgliche Salze mit Säuren und Basen.

3. Verfahren gemäß Anspruch 1 oder 2, dadurch gekennzeichnet, daß eine Verbindung der Formel I hergestellt wird in welcher

n = 1 oder 2 ist

R = Wasserstoff,

Alkyl mit 1 - 8 C-Atomen,

Alkenyl mit 2 - 6 C-Atomen,

Cycloalkyl mit 3 - 9 C-Atomen,

Aryl mit 6 - 12 C-Atomen,
das durch ($C_1$-$C_4$)-Alkyl, ($C_1$-$C_4$)-Alkoxy, Hydroxy, Halogen, Nitro, Amino, Aminomethyl, ($C_1$-$C_4$)-Alkylamino, di-($C_1$-$C_4$)-alkylamino, ($C_1$-$C_4$)-Alkanoylamino, Methylendioxy, Carboxy, Cyano und/oder Sulfamoyl, mono-, di- oder trisubstituiert sein kann,
Alkoxy mit 1 - 4 C-Atomen,
Aryloxy mit 6 - 12 C-Atomen,
das wie oben bei Aryl beschrieben substituiert sein kann,
mono- bzw. bicyclisches Heteroaryloxy mit 5 - 7 bzw. 8 - 10 Ringatomen, wovon 1 bis 2 Ringatome Schwefel-oder Sauerstoffatome und/oder 1 bis 4 Ringatome Stickstoff darstellen,
das wie oben bei Aryl beschrieben substituiert sein kann,
Amino-($C_1$-$C_4$)-alkyl,
($C_1$-$C_4$)-Alkanoylamino-($C_1$-$C_4$)alkyl,
($C_7$-$C_{13}$)-Aroylamino-($C_1$-$C_4$)-alkyl,
($C_1$-$C_4$)-Alkoxy-carbonylamino-($C_1$-$C_4$)-alkyl,
($C_6$-$C_{12}$)-Aryl-($C_1$-$C_4$)-alkoxycarbonylamino-($C_1$-$C_4$)-alkyl,
($C_6$-$C_{12}$)-aryl-($C_1$-$C_4$)-alkylamino-($C_1$-$C_4$)-alkyl,
($C_1$-$C_4$)-Alkylamino-($C_1$-$C_4$)-alkyl,
Di-($C_1$-$C_4$)-alkylamino-($C_1$-$C_4$)-alkyl,
Guanidino-($C_1$-$C_4$)-alkyl,
Imidazolyl, Indolyl,
($C_1$-$C_4$)-Alkylthio,
($C_1$-$C_4$)-Alkylthio-($C_1$-$C_4$)-alkyl,
($C_6$-$C_{12}$)-Arylthio-($C_1$-$C_4$)-alkyl,
das im Arylteil wie oben bei Aryl beschrieben, substituiert sein kann,
($C_6$-$C_{12}$)-Aryl-($C_1$-$C_4$)-alkylthio,
das im Arylteil wie oben bei Aryl beschrieben substituiert sein kann,
Carboxy-($C_1$-$C_4$)-alkyl,
Carboxy, Carbamoyl,
Carbamoyl-($C_1$-$C_4$)-alkyl,
($C_1$-$C_4$)-alkoxy-carbonyl-($C_1$-$C_4$)-alkyl,
($C_6$-$C_{12}$)-Aryloxy-($C_1$-$C_4$)-alkyl,
das im Arylteil wie oben bei Aryl beschrieben substituiert sein kann oder
($C_6$-$C_{12}$)-Aryl-($C_1$-$C_4$)-alkoxy,
das im Arylteil wie oben bei aryl beschrieben substituiert sein kann,
$R^1$ Wasserstoff,
Alkyl mit 1 - 6 C-Atomen,
Alkenyl mit 2 - 6 C-Atomen,
Alkinyl mit 2 - 6 C-Atomen,
Cycloalkyl mit 3 - 9 C-Atomen,
Cycloalkenyl mit 5 - 9 C-Atomen,
($C_3$-$C_9$)-Cycloalkyl-($C_1$-$C_4$)-alkyl,
($C_5$-$C_9$)-Cycloalkenyl-($C_1$-$C_4$)-alkyl,
gegebenenfalls teilhydriertes Aryl mit 6 - 12 C-Atomen, das wie oben bei R beschrieben substituiert sein kann, ($C_6$-$C_{12}$)-Aryl-($C_1$-$C_4$)-alkyl oder ($C_7$-$C_{13}$)-Aroyl-($C_1$ oder $C_2$)-alkyl
die beide wie das vorstehende Aryl substituiert sein können
mono- bzw. bicyclisches, gegebenenfalls teilhydriertes Heteroaryl mit 5 - 7 bzw. 8 - 10 Ringatomen, wovon 1 bis 2 Ringatome Schwefel- oder Sauerstoffatome und/oder
1 bis 4 Ringatome Stickstoffatome darstellen, das wie das vorstehende Aryl substituiert sein kann oder
die gegebenenfalls geschützte Seitenkette einer natürlichen vorkommenden α-Aminosäure $R^1$-$CH(NH_2)$-COOH bedeutet,
$R^2$ und $R^3$ gleich oder verschieden sind und
Wasserstoff,
Alkyl mit 1 - 6 C-Atomen,
Alkenyl mit 2 - 6 C-Atomen,
Di-($C_1$-$C_4$)-alkylamino($C_1$-$C_4$)-alkyl,
($C_1$-$C_5$)-Alkanoyloxy-($C_1$-$C_4$)-alkyl,
($C_1$-$C_5$)-Alkoxy-carbonyloxy-($C_1$-$C_4$)-alkyl,
($C_7$-$C_{13}$)-Aroyloxy-($C_1$-$C_4$)-alkyl,

$(C_6-C_{12})$-Aryloxycarbonyloxy$(C_1-C_4)$-alkyl,
Aryl mit 6 - 12 C-Atomen,
$(C_6-C_{12})$-Aryl-$(C_1-C_4)$-alkyl,
$(C_3-C_9)$-Cycloalkyl,
$(C_3-C_9)$-Cycloalkyl-$(C_1-C_4)$-alkyl,
Phthalidyl,
einen Rest der Formel

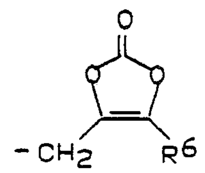

worin $R^6$ Wasserstoff, $(C_1-C_6)$-Alkyl oder Aryl mit 6 - 12 C-Atomen ist,
einen Rest der Formel

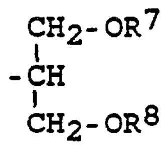

bedeuten, worin $R^7$ und $R^8$ gleich oder verschieden unabhängig voneinander Wasserstoff oder einen gegebenenfalls substituierten, unverzweigten oder verzweigten, gesättigten oder ungesättigten Acylrest mit 1 - 23 C-Atomen bedeuten,
und wobei mindestens einer der Reste $R^2$ oder $R^3$ einen Rest der Formel

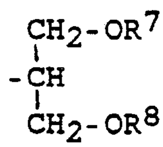

bedeutet, und
$R^4$ und $R^5$ die oben angegebene Bedeutung haben oder deren physiologisch verträgliche Salze mit Säuren und Basen.

4. Verfahren gemäß einem oder mehrerer der Ansprüche 1 bis 3, dadurch gekennzeichnet, daß eine Verbindung der Formel I hergestellt wird, in welcher
n = 1 oder 2 ist,
R $(C_1-C_6)$-Alkyl, $(C_2-C_6)$-Alkenyl, $(C_3-C_9)$-Cycloalkyl, Amino-$(C_1-C_4)$-alkyl, $(C_2-C_5)$-Acylamino-$(C_1-C_4)$-alkyl, $(C_7-C_{13})$-Aroylamino-$(C_1-C_4)$-alkyl, $(C_1-C_4)$-Alkoxy-carbonylamino-$(C_1-C_4)$-alkyl, $(C_6-C_{12})$-Aryl-$(C_1-C_4)$-alkoxycarbonylamino-$(C_1-C_4)$-alkyl, $(C_6-C_{12})$-Aryl, das durch $(C_1-C_4)$-Alkyl, $(C_1-C_4)$-Alkoxy, Hydroxy, Halogen, Nitro, Amino, $(C_1-C_4)$-Alkylamino, Di-$(C_1-C_4)$-alkylamino und/oder Methylendioxy mono-, di- oder trisubstituiert sein kann, oder 3-Indolyl, insbesondere Methyl, Ethyl, Cyclohexyl, tert. Butoxycarbonylamino-$(C_1-C_4)$-alkyl, Benzoyloxycarbonylamino-$(C_1-C_4)$-alkyl oder Phenyl, das durch Phenyl, $(C_1-C_2)$-Alkyl, ($C_1$ oder $C_2$)-Alkoxy, Hydroxy, Fluor, Chlor, Brom, Amino, $(C_1-C_4)$-Alkylamino, Di-$(C_1-C_4)$alkylamino, Nitro und/oder Methylendioxy mono- oder disubstituiert oder im Falle von Methoxy, trisubstituiert sein kann, bedeutet,
$R^1$ Wasserstoff oder $(C_1-C_6)$-Alkyl, das gegebenenfalls durch Amino, $(C_1-C_6)$-Acylamino oder Benzoylamino substituiert sein kann, $(C_2-C_6)$-Alkenyl, $(C_3-C_9)$-Cycloalkyl, $(C_5-C_9)$-Cycloakenyl, $(C_3-C_7)$-Cycloalkyl-$(C_1-C_4)$-alkyl, $(C_6-C_{12})$-Aryl oder teilhydriertes Aryl, das jeweils durch $(C_1-C_4)$-Alkyl, ($C_1$ oder $C_2$)-Alkoxy oder Halogen substituiert sein kann, $(C_6-C_{12})$-Aryl-($C_1$ bis $C_4$)-alkyl oder $(C_7-C_{13})$-Aroyl-$(C_1-C_2)$-alkyl, die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder

Sauerstoffatome und/oder 1 bis 4 Ringatome Stickstoffatome darstellen, oder eine Seitenkette einer natürlich vorkommenden, gegebenenfalls geschützten α-Aminosäure, insbesondere aber Wasserstoff, ($C_1$-$C_3$)-Alkyl, ($C_2$ oder $C_3$)-Alkenyl, die gegebenenfalls geschützte Seitenkette von Lysin, Phenylalanin oder Tyrosin, Benzyl, 4-Methoxybenzyl, 4-Ethoxybenzyl, Phenethyl, 4-Amino-butyl oder Benzoylmethyl bedeutet,

$R^2$ und $R^3$ gleiche oder verschiedene Reste Wasserstoff, ($C_1$-$C_6$)-Alkyl, ($C_2$-$C_6$)-Alkenyl, ($C_6$-$C_{12}$)-Aryl-($C_1$-$C_4$)-alkyl, ($C_1$-$C_5$)-Alkanoyloxy-($C_1$-$C_4$)-alkyl, ($C_1$-$C_6$)-Alkoxycarbonyloxy-($C_1$-$C_4$)-alkyl, ($C_7$-$C_{13}$)-Aroyloxy-($C_1$-$C_4$)-alkyl, ($C_6$-$C_{12}$)-Aryloxycarbonyloxy-($C_1$-$C_4$)alkyl, Phthalidyl, einen Rest der Formel

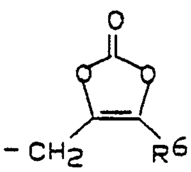

worin $R^5$ Wasserstoff, ($C_1$-$C_6$)-Alkyl oder Aryl mit 6 - 12 C-Atomen ist, oder einen Rest der Formel

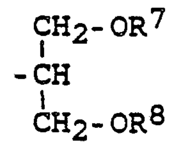

worin $R^7$ und $R^8$ Wasserstoff oder einen gesättigten oder ungesättigten Acylrest mit 8 - 23 C-Atomen bedeuten,
und wobei mindestens einer der Reste $R^2$ oder $R^3$ einen Rest der Formel

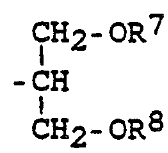

bedeutet und
$R^4$ und $R^5$ die oben angegebene Bedeutung haben oder deren physiologisch verträgliche Salze mit Säuren und Basen.

5. Verwendung einer Verbindung gemäß einem oder mehrerer der Ansprüche 1 bis 4 zur Herstellung eines Heilmittels für die Behandlung des Bluthochdrucks.

6. Verwendung einer Verbindung gemäß einem oder mehrerer der Ansprüche 1 bis 4 zur Herstellung eines Heilmittels für die Behandlung cognitiver Dysfunktionen.

7. Verfahren zur Herstellung eines pharmazeutisches Mittel enthaltend eine Verbindung gemäß einem oder mehrerer der Ansprüche 1 bis 4, dadurch gekennzeichnet, daß man eine Verbindung der Formel I oder deren physiologisch verträgliches Salz und einen physiologisch annehmbaren Träger gegebenenfalls zusammen mit weiteren Zusatz- oder Hilfsstoffen in eine geeignete Darreichungsform bringt.